# EUROPEAN PATENT SPECIFICATION

(11) **EP 4 061 358 B1**
(45) Date of publication and mention of the grant of the patent: **28.05.2025**
(21) Application number: 20888950.1
(22) Date of filing: 20.11.2020
(51) Int. Cl.: A61K 31/403, A61P 1/00, A61P 1/04, C07D 209/94, A61K 31/404, A61K 9/20

(54) **ETRASIMOD FOR USE IN THE TREATMENT OF EOSINOPHILIC ESOPHAGITIS**
ETRASIMOD ZUR VERWENDUNG IN DER BEHANDLUNG VON EOSINOPHILER ÖSOPHAGITIS
ETRASIMOD DESTINÉ À ÊTRE UTILISÉ DANS LE TRAITEMENT DE L' OESOPHAGITE À ÉOSINOPHILES

(30) Priority: 20.11.2019 US 201962938008 P
(43) Date of publication of application: 28.09.2022
(73) Proprietor: Arena Pharmaceuticals, Inc., New York, NY 10001-2192 (US)
(72) Inventor: KOMORI-OCCHICONE, Heather, Kiyomi, San Diego, CA 92129 (US); NGUYEN-CLEARY, Thai, Curtis, Carlsbad, CA 92009 (US); YU, Jin, Tuscon, AZ 85045 (US)
(74) Representative: Pfizer
(86) International application number: PCT/US2020/061653
(87) International publication number: WO 2021/102357

(56) References cited:
- WO-A1-2012/061459
- US-A1- 2019 135 752
- US-B2- 7 759 370
- GONSALVES NIRMALA ED - BALLOW MARK: "Eosinophilic Gastrointestinal Disorders", CLINICAL REVIEWS IN ALLERGY AND IMMUNOLOGY, HUMANA PRESS, TOTOWA, NJ, US, vol. 57, no. 2, 22 March 2019 (2019-03-22), pages 272 - 285, XP036907337, ISSN: 1080-0549, [retrieved on 20190322], DOI: 10.1007/S12016-019-08732-1
- DELLON EVAN S. ET AL: "Efficacy and Safety of the Selective Sphingosine 1-Phosphate Receptor Modulator, Etrasimod, in Adult Patients With Eosinophilic Esophagitis: Primary Results From the Phase 2 VOYAGE Study", AMERICAN JOURNAL OF GASTROENTEROLOGY, vol. 118, no. 10S, 1 October 2023 (2023-10-01), US, pages S330 - S331, XP093209659, ISSN: 0002-9270, DOI: 10.14309/01.ajg.0000951460.86738.5a
- CROSBY C M; KOMORI H K; ADAMS J W: "Etrasimod, an Oral, Selective Sphingosine 1-phosphate Receptor Modulator Improves Skin Inflammation in a Contact Hypersensitivity Model of Dermatitis", POSTER #30, 18 September 2019 (2019-09-18), Bordeaux France, XP009517577
- KONIKOFF ET AL.: "Potential of Blood Eosinophils, Eosinophil-derived Neurotoxin, and Eotaxin-3 as Biomarkers of Eosinophilic Esophagitis", CLINICAL GASTROENTEROLOGY AND HEPTALOGY, vol. 4, 2006, pages 1328 - 1336, XP005725858, DOI: 10.1016/j.cgh.2006.08.013

## Description

Provided is a compound for use in a method of treatment of eosinophilic esophagitis. See, e.g., Gonsalves Clin Rev Allergy Immunol https://doi.org/10.1007/s12016-019-08732-1.

Esophageal inflammation disorders such as eosinophilic esophagitis (EoE), a disease characterized by high levels of eosinophils in the esophagus, as well as basal zonal hyperplasia, is increasingly being diagnosed in children and adults. Many aspects of the disease remain unclear including its etiology, natural history, and optimal therapy. EoE affects all age groups but most frequently individuals between 20 and 50 years of age. Symptoms of EoE often mimic those of gastroesophageal reflux disease (GERD) and include vomiting, dysphagia, pain, and food impaction. The disease is painful, leads to difficulty swallowing, and predisposes patients to other complications. EoE is often misdiagnosed for GERD, causing delay in adequate treatment for EoE patients.

Diagnostic criteria currently required for the diagnosis of EoE include 1) symptoms of esophageal dysfunction; 2) eosinophilic esophageal inflammation with at least 15 eosinophils per high-power field affecting the esophagus alone; and 3) exclusion of other causes of esophageal eosinophilia. The other causes may include eosinophilic gastroenteritis, celiac disease, Crohn's disease (CD), infection, achalasia, vasculitis, and hypereosinophilic syndrome.

Though the etiology of EoE is not yet completely understood, EoE is considered a type 2 helper T (Th2) cell-mediated atopic disease. EoE onset is thought to arise from a multifactorial interaction between genetic susceptibility and inappropriate immune-driven inflammatory responses to food antigens (predominantly non-IgE-mediated), aeroallergens, and environmental factors. A genetic predisposition to disturbed barrier function, which is observed in esophageal tissue from EoE patients, permits allergenic molecules easy entry through the epithelium and subsequent Th2-driven allergic hypersensitivity. This Th2 cell-mediated activity leads to cytokine production (including interleukin [IL]-4, IL-5, and IL-13) and subsequent eosinophil activation and recruitment to the esophagus (eosinophilic inflammation). Once in the esophagus, eosinophils release harmful secretory products that cause the previously described esophageal symptoms, tissue damage and remodeling, and elicits additional barrier disruption, further reinforcing the inflammatory cycle.

There remains a great unmet clinical need for new efficacious and safe treatments for EoE, as current therapies often provide only transient or marginal symptomatic relief. The present disclosure satisfies this need and provides related advantages as well.

Citation of any reference throughout this application is not to be construed as an admission that such reference is prior art to the present application.

WO-2012/061459 discloses compounds said to be S1P₁ agonists and therefore useful for the treatment of autoimmune and vascular disorders including eosinophilic gastroenteritis.

Crosby et al, (Poster #30, 18.09.2019, Bordeaux, France) describe the compound etrasimod as an oral, selective sphingosine-1-phosphate receptor modulator that improves skin inflammation in a contact hypersensitivity model of dermatitis.

### SUMMARY

Provided is a compound that is (*R*)-2-(7-(4-cyclopentyl-3-(trifluoromethyl)benzyloxy)-1,2,3,4-tetrahydrocyclopenta[*b*]indol-3-yl)acetic acid (Compound 1), or a pharmaceutically acceptable salt thereof, for use in a method of treatment of eosinophilic esophagitis (EoE) in an individual, wherein the method comprises administering to an individual in need thereof a therapeutically effective amount of the compound or salt thereof.
In some embodiments, the individual in need thereof exhibits ≥15 eosinophils per high powered field (hpf) in the individual's esophagus prior to or at the time of the treatment ("baseline"). In some embodiments, the individual in need thereof exhibits at least 30% decrease in the number of eosinophils per hpf from baseline at week 16 following the administration of Compound 1, or a pharmaceutically acceptable salt thereof. According to some embodiments, the Compound 1, or a pharmaceutically acceptable salt thereof is administered under fasted conditions. According to some embodiments, the Compound 1, or a pharmaceutically acceptable salt thereof is administered under fed conditions. In some embodiments, the therapeutically effective amount is equivalent to about 0.5 to about 5.0 mg of Compound 1. In some embodiments, the therapeutically effective amount is in an amount equivalent to 2 mg of Compound 1. In some embodiments, the therapeutically effective amount is in an amount equivalent to 1 mg of Compound 1. According to some embodiments, the therapeutically effective amount is administered at a frequency of once per day. In some embodiments, the therapeutically effective amount is administered in the morning. In some embodiments, the individual exhibits an esophageal PEC of less than 15 eos/hpf from baseline following the administration of Compound 1, or a pharmaceutically acceptable salt thereof. In some embodiments, the individual exhibits an esophageal PEC of less than 6 eos/hpf from baseline following the administration of Compound 1, or a pharmaceutically acceptable salt thereof. In some embodiments, the individual exhibits at least 50% decrease in the number of eosinophils per hpf from baseline at week 16 following the administration of Compound 1, or a pharmaceutically acceptable salt thereof. In some embodiments, the individual exhibits at least 40% decrease in the number of eosinophils per hpf from baseline at week 16 following the administration of Compound 1, or a pharmaceutically acceptable salt thereof. In some embodiments, individual exhibits at least 50% decrease in the number of eosinophils per hpf from baseline at week 16 following the administration of Compound 1, or a pharmaceutically acceptable salt thereof. According to some embodiments, the administering results in no serious adverse events. In some embodiments, the Compound 1, or a pharmaceutically acceptable salt thereof, is administered without substantially inducing an acute heart rate reduction or heart block in the individual.

These and other aspects of the invention disclosed herein will be set forth in greater detail as the patent disclosure proceeds.

### BRIEF DESCRIPTION OF DRAWINGS

**Figure 1**: The pathogenesis of EoE is shown. In step 1, esophageal epithelial cells polarize dendritic cells to a Th2 phenotype. In step 2, dendritic cells migrate to a lymph node (LN) and promote Th2 T cell differentiation. In step 3, newly activated Th2 cells leave the LN. In step 4, Th2 cells migrate to the esophagus and secrete cytokines. In step 5, eosinophils are recruited to the esophagus via the Th2 cytokines.
**Figure 2****:** Endoscopic manifestations in adult EoE patients enrolled in a European multicenter trial are shown: white exudate (a), longitudinal furrows (b), diffuse edema (c), fixed rings (d), severe stricture (e), and rings, furrows and edema (f).
**Figure 3** shows the effect of Compound 1 on the mean percent change from baseline to day 7 in immune cell subtypes, as further described in Example 3.

### DETAILED DESCRIPTION

As used in the present specification, the following words and phrases are generally intended to have the meanings as set forth below, except to the extent that the context in which they are used indicates otherwise.

**COMPOUND 1**: As used herein, "Compound 1" means (R)-2-(7-(4-cyclopentyl-3-(trifluoromethyl)benzyloxy)-1,2,3,4-tetrahydrocyclopenta[b]indol-3-yl)acetic acid including crystalline forms thereof. 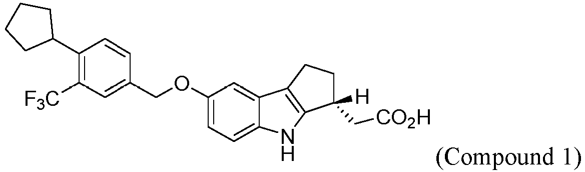 See PCT patent application, Serial No. PCT/US2009/004265 in its entirety. As a non-limiting example, Compound 1 may be present as an anhydrous, non-solvated crystalline form as described in WO 2010/011316. As another non-limiting example, an L-arginine salt of Compound 1 may be present as an anhydrous, non-solvated crystalline form as described in WO 2010/011316 and WO 2011/094008. As another non-limiting example, a calcium salt of Compound 1 may be present as a crystalline form as described in WO 2010/011316. Compound 1 is referred to in literature as etrasimod or APD334.

Compound 1, or a pharmaceutically acceptable salt, solvate, or hydrate thereof, is an orally administered, selective, synthetic sphingosine 1-phosphate (S1P) receptor 1, 4, 5 modulator. To date, Compound 1, or a pharmaceutically acceptable salt, solvate, or hydrate thereof, has been found to be safe and well-tolerated in approximately 281 adult subjects treated at various doses. Its safety and tolerability have been evaluated in Phase 1 studies with healthy adult subjects at single doses up to 5 mg and repeated doses up to 4 mg once daily (QD). In a Phase 2 dose-ranging study in UC patients, treatment with 2 mg QD for 12 weeks led to clinically meaningful and statistically significant endoscopic and symptomatic improvements versus placebo. Sustained beneficial effects of were observed for up to 46 weeks in the subsequent open-label extension study.

**ADMINISTERING:** As used herein, "administering" means to provide a compound or other therapy, remedy, or treatment such that an individual internalizes a compound.

**PRESCRIBING:** As used herein, "prescribing" means to order, authorize, or recommend the use of a drug or other therapy, remedy, or treatment. In some embodiments, a health care practitioner can orally advise, recommend, or authorize the use of a compound, dosage regimen or other treatment to an individual. In this case the health care practitioner may or may not provide a prescription for the compound, dosage regimen, or treatment. Further, the health care practitioner may or may not provide the recommended compound or treatment. For example, the health care practitioner can advise the individual where to obtain the compound without providing the compound. In some embodiments, a health care practitioner can provide a prescription for the compound, dosage regimen, or treatment to the individual. For example, a health care practitioner can give a written or oral prescription to an individual. A prescription can be written on paper or on electronic media such as a computer file, for example, on a hand-held computer device. For example, a health care practitioner can transform a piece of paper or electronic media with a prescription for a compound, dosage regimen, or treatment. In addition, a prescription can be called in (oral), faxed in (written), or submitted electronically via the internet to a pharmacy or a dispensary. In some embodiments, a sample of the compound or treatment can be given to the individual. As used herein, giving a sample of a compound constitutes an implicit prescription for the compound. Different health care systems around the world use different methods for prescribing and/or administering compounds or treatments and these methods are encompassed by the disclosure.

A prescription can include, for example, an individual's name and/or identifying information such as date of birth. In addition, for example, a prescription can include: the medication name, medication strength, dose, frequency of administration, route of administration, number or amount to be dispensed, number of refills, physician name, physician signature, and the like. Further, for example, a prescription can include a DEA number and/or state number.

A healthcare practitioner can include, for example, a physician, nurse, nurse practitioner, or other related health care professional who can prescribe or administer compounds (drugs) for the treatment of a condition described herein. In addition, a healthcare practitioner can include anyone who can recommend, prescribe, administer, or prevent an individual from receiving a compound or drug including, for example, an insurance provider.

**PREVENT, PREVENTING, OR PREVENTION:** As used herein, the term "prevent," "preventing", or "prevention," such as prevention of a particular disorder or the occurrence or onset of one or more symptoms associated with the particular disorder and does not necessarily mean the complete prevention of the disorder. For example, the term "prevent," "preventing" and "prevention" means the administration of therapy on a prophylactic or preventative basis to an individual who may ultimately manifest at least one symptom of a disease or condition but who has not yet done so. Such individuals can be identified on the basis of risk factors that are known to correlate with the subsequent occurrence of the disease. Alternatively, prevention therapy can be administered without prior identification of a risk factor, as a prophylactic measure. Delaying the onset of at least one symptom can also be considered prevention or prophylaxis.

**TREAT, TREATING, OR TREATMENT:** As used herein, the term "treat," "treating", or "treatment" means the administration of therapy to an individual who already manifests at least one symptom of a disease or condition or who has previously manifested at least one symptom of a disease or condition. For example, "treating" can include alleviating, abating or ameliorating a disease or condition symptoms, preventing additional symptoms, ameliorating the underlying metabolic causes of symptoms, inhibiting the disease or condition, e.g., arresting the development of the disease or condition, relieving the disease or condition, causing regression of the disease or condition, relieving a condition caused by the disease or condition, or stopping the symptoms of the disease or condition. For example, the term "treating" in reference to a disorder means a reduction in severity of one or more symptoms associated with that particular disorder. Therefore, treating a disorder does not necessarily mean a reduction in severity of all symptoms associated with a disorder and does not necessarily mean a complete reduction in the severity of one or more symptoms associated with a disorder.

**TOLERATE:** As used herein, an individual is said to "tolerate" a dose of a compound if administration of that dose to that individual does not result in an unacceptable adverse event or an unacceptable combination of adverse events. One of skill in the art will appreciate that tolerance is a subjective measure and that what may be tolerable to one individual may not be tolerable to a different individual. For example, one individual may not be able to tolerate headache, whereas a second individual may find headache tolerable but is not able to tolerate vomiting, whereas for a third individual, either headache alone or vomiting alone is tolerable, but the individual is not able to tolerate the combination of headache and vomiting, even if the severity of each is less than when experienced alone.

**INTOLERANCE:** As used herein, "intolerance" means significant toxicities and/or tolerability issues that led to a reduction in dose or discontinuation of the medication. "Intolerance" can be replaced herein with the term "unable to tolerate."

**ADVERSE EVENT:** As used herein, an "adverse event" is an untoward medical occurrence that is associated with treatment with Compound 1 or a pharmaceutically acceptable salt, solvate, or hydrate thereof. In one embodiment, an adverse event is selected from: leukopenia, constipation, diarrhea, nausea, abdominal pain, neutropenia, vomiting, back pain, and menstrual disorder. In one embodiment, an adverse event is heart block, for example, a first-degree atrioventricular heart block. In one embodiment, an adverse event is an acute heart rate reduction. In one embodiment, an adverse event is an abnormal pulmonary function test finding, such as an FEV1 below 80%, FVC. In one embodiment, an adverse event is an abnormal liver function test, such as an elevated ALT & AST>2X ULN. In one embodiment, an adverse event is macular edema.

**IN NEED OF TREATMENT** and **IN NEED THEREOF:** As used herein, "in need of treatment" and "in need thereof" when referring to treatment are used interchangeably to mean a judgment made by a caregiver (*e.g.* physician, nurse, nurse practitioner, *etc.*) that an individual requires or will benefit from treatment. This judgment is made based on a variety of factors that are in the realm of a caregiver's expertise, but that includes the knowledge that the individual is ill, or will become ill, as the result of a disease, condition or disorder that is treatable by the compounds of the invention. Accordingly, the compounds of the invention can be used in a protective or preventive manner; or compounds of the invention can be used to alleviate, inhibit or ameliorate the disease, condition or disorder.

**INDIVIDUAL:** As used herein, "individual" means any human. In some embodiments, a human individual is referred to a "subject" or "patient."

**ACUTE HEART RATE REDUCTION:** As used herein, "acute heart rate reduction" means a heart rate decrease from normal sinus rhythm of, for example, 10 or more beats per minute (bpm), such as less than about 5 bpm, *e.g.,* less than about 4 bpm or less than about 3 bpm or less than 2 bpm, that is maximal within a few hours, for example 1-3 hours, after drug administration, and thereafter the heart rate returns towards the pre-dose value.

**NORMAL SINUS RHYTHM:** As used herein, "normal sinus rhythm" means the sinus rhythm of the individual when not undergoing treatment. The evaluation of normal sinus rhythm is within the ability of a physician. A normal sinus rhythm will generally give rise to a heart rate in the range from 60-100 bpm.

**DOSE:** As used herein, "dose" means a quantity of Compound 1, or a pharmaceutically acceptable salt, solvate, or hydrate thereof, given to the individual for treating or preventing the disease or disorder at one specific time.

**STANDARD DOSE:** As used herein, "standard dose" means the dose of Compound 1, or a pharmaceutically acceptable salt, solvate, or hydrate thereof, that is given to the individual for treating or preventing the disease or disorder. The target dose may vary depending on the nature and severity of the disease to be treated.

**THERAPEUTICALLY EFFECTIVE AMOUNT:** As used herein, "therapeutically effective amount" of an agent, compound, drug, composition or combination is an amount which is nontoxic and effective for producing some desired therapeutic effect upon administration to a subject or patient (e.g., a human subject or patient). The precise therapeutically effective amount for a subject may depend upon, e.g., the subject's size and health, the nature and extent of the condition, the therapeutics or combination of therapeutics selected for administration, and other variables known to those of skill in the art. The effective amount for a given situation is determined by routine experimentation and is within the judgment of the clinician. In some embodiments, the therapeutically effective amount is the standard dose.

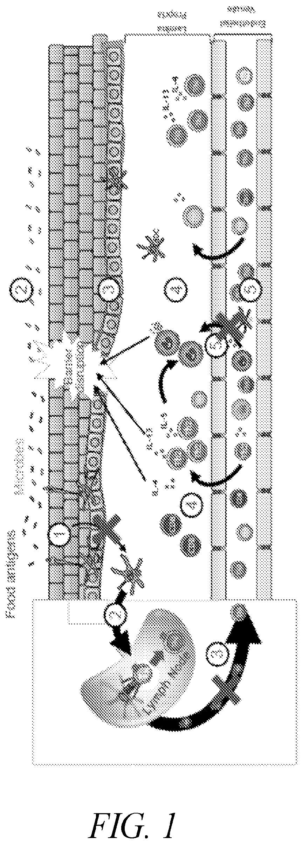

**EOSINOPHILIC ESOPHAGITIS:** As used herein, "eosinophilic esophagitis" or "EoE" means an inflammatory disease characterized by abnormal eosinophilic inflammation within the esophagus and esophageal dysfunction. The pathogenesis of EoE is shown in Figure 1. The primary symptoms of EoE include, but are not limited to, chest and abdominal pain, dysphagia, heartburn, food refusal, vomiting and food impaction. The clinicopathology of EoE is characterized by presence of ridges or trachea-like rings in the esophageal wall and eosinophilic infiltration in the esophageal mucosa. EoE is presently diagnosed by endoscopy of the esophagus followed by microscopic and biochemical analysis of the esophageal mucosal lining. EoE may be classified as allergic or non-allergic depending upon the status of the subject. The present invention includes methods to treat both allergic and non-allergic forms of EoE.

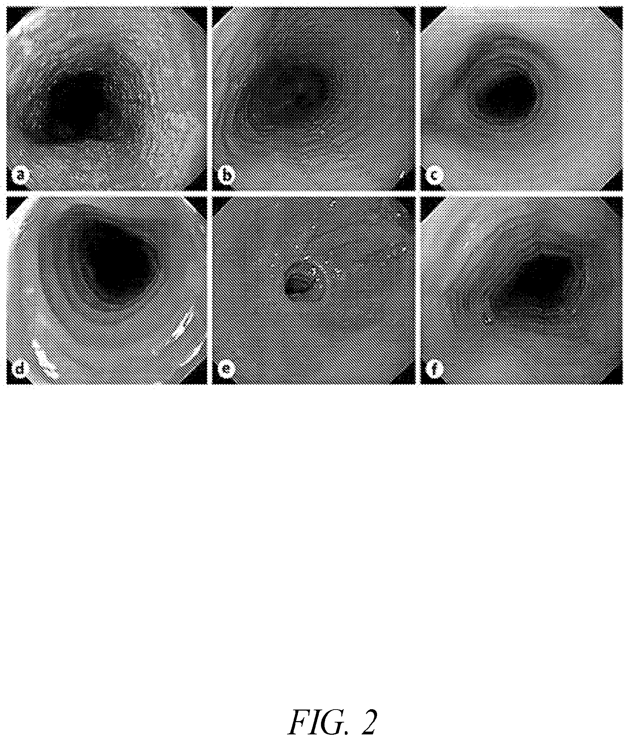

**ESOPHAGEAL STRICTURES:** As used herein, esophageal strictures can be classified as simple or complex, based on their diameter and associated anatomic abnormalities. A simple stricture is defined as a short stricture with a symmetric or concentric lumen and a diameter of ≥12 mm that can be traversed easily with an endoscope. A complex stricture is usually longer than 2 cm, may be angulated or irregular, and has a diameter of <12 mm. It may be associated with a large hiatal hernia, esophageal diverticula, or tracheoesophageal fistula. Complex strictures have a higher rate of recurrence and an increased risk for dilation-related adverse events, compared with simple strictures. The severity of a stricture can be estimated by the resistance encountered with passage of the diagnostic endoscope, which has a typical external diameter of 9 mm. A mild stricture allows passage of the endoscope without resistance, a moderate stricture offers increased resistance, whereas a severe stricture may not be traversable. See **Figure 2****.**

**ALLERGEN:** As used herein, "allergen," means any substance, chemical, particle or composition which is capable of stimulating an allergic response in a susceptible individual. Allergens may be contained within or derived from a food item such as, e.g., dairy products (e.g., cow's milk), egg, wheat, soy, corn, rye, fish, shellfish, peanuts and tree nuts. Alternatively, an allergen may be contained within or derived from a non-food item such as, e.g., dust (e.g., containing dust mite), pollen, insect venom (e.g., venom of bees, wasps, mosquitoes, etc.), mold, animal dander, latex, medication, drugs, ragweed, grass and birch.

**ALLERGIC RESPONSE** or **ALLERGIC REACTION** or **ALLERGIC SYMPTOM:** As used herein, the phrases "allergic response," "allergic reaction," "allergic symptom," and the like, include one or more signs or symptoms selected from urticaria (e.g., hives), angioedema, rhinitis, asthma, vomiting, sneezing, runny nose, sinus inflammation, watery eyes, wheezing, bronchospasm, reduced peak expiratory flow (PEF), gastrointestinal distress, flushing, swollen lips, swollen tongue, reduced blood pressure, anaphylaxis, and organ dysfunction/failure. An "allergic response," "allergic reaction," "allergic symptom," etc., also includes immunological responses and reactions such as, e.g., increased IgE production, increased allergen-specific immunoglobulin production and/or eosinophilia.

**EOSINOPHILIC INFILTRATION:** As used herein, "eosinophilic infiltration" refers to the presence of eosinophils in an organ or tissue including blood, esophagus, stomach, duodenum, and ileum of a subject and more specifically, to presence of eosinophils in the mucosal lining of a region of the gastro-intestinal tract including, but not limited to, esophagus and stomach. Eosinophilic infiltration is analyzed, for example, in an esophageal tissue biopsy of a subject suffering from EoE. According to some embodiments, "eosinophilic infiltration" refers to the presence of ≥15 eosinophils per high power field in the esophagus. The term "high power field" refers to a standard total magnification of 400x by a microscope used to view eosinophils in a tissue, e.g., from the esophagus of a subject. In certain embodiments, "eosinophilic infiltration" includes infiltration into a tissue by leucocytes, for example, lymphocytes, neutrophils and mast cells. The leucocyte infiltration into, e.g., esophageal tissue can be detected by cell surface markers such as eosinophil-specific markers (e.g., CD11c^{Low/Neg}, SiglecF⁺, F4/80⁺, EMR1⁺, Siglec 8⁺, and MBP2⁺), macrophage-specific markers (e.g., CD11b⁺, F4/80⁺, CD14⁺, EMR1⁺, and CD68⁺), neutrophil-specific markers (e.g., CD11b⁺, Ly6G⁺, Ly6C⁺, CD11b⁺, and CD66b⁺), and T-cell-specific markers (e.g., CD3⁺ CD4⁺ CD8⁺).

**REDUCTION IN ESOPHAGUS EOSINOPHILS:** As used herein, "a reduction in esophagus eosinophils" means that the number of eosinophils and other leucocytes measured in the esophagus of a subject with EoE and who has been treated with Compound 1, or a pharmaceutically acceptable salt or as a solvate or hydrate thereof, is at least 5%, 10%, 20%, 50%, 70%, 80%, or 90% lower than the esophagus eosinophils measured in the same or an equivalent subject that has not been treated with Compound 1, or a pharmaceutically acceptable salt or as a solvate or hydrate thereof. In certain embodiments, reducing eosinophilic infiltration means detecting less than 15 eosinophils per high power field, such as less than 10 eosinophils, less than 9 eosinophils, less than 8 eosinophils, less than 7 eosinophils, less than 6 eosinophils, or less than 5 eosinophils per high power field in a biopsy of the esophageal mucosa. In certain embodiments, a reduction in esophagus eosinophils means that no eosinophils are detected in the esophageal mucosa of a subject.

**EOE-ASSOCIATED BIOMARKER:** As used herein, the term "EoE-associated biomarker" means any biological response, cell type, parameter, protein, polypeptide, enzyme, enzyme activity, metabolite, nucleic acid, carbohydrate, or other biomolecule which is present or detectable in an EoE patient at a level or amount that is different from (e.g., greater than or less than) the level or amount of the marker present or detectable in a non-EoE patient. Exemplary EoE-associated biomarkers include, but are not limited to, e.g., esophagus eosinophils, eotaxin-3 (CCL26), periostin, serum IgE (total and allergen-specific), IL-13, IL-5, serum thymus and activation regulated chemokine (TARC; CCL17), thymic stromal lymphopoietin (TSLP), serum eosinophilic cationic protein (ECP), and eosinophil-derived neurotoxin (EDN). The term "EoE-associated biomarker" also includes a gene or gene probe known in the art which is differentially expressed in a subject with EoE as compared to a subject without EoE. For example, genes which are significantly up-regulated in a subject with EoE include, but are not limited to, T-helper 2 (Th2)-associated chemokines such as CCL8, CCL23 and CCL26, periostin, cadherin-like-26, and TNFα-induced protein 6. Alternatively, "EoE-associated biomarker" also includes genes which are downregulated due to EoE such as terminal differentiation proteins (e.g., filaggrin). Certain embodiments relate to use of these biomarkers for monitoring disease reversal with the administration of Compound 1, or a pharmaceutically acceptable salt or as a solvate or hydrate thereof. Methods for detecting and/or quantifying such EoE-associated biomarkers are known in the art; kits for measuring such EoE-associated biomarkers are available from various commercial sources; and various commercial diagnostic laboratories offer services which provide measurements of such biomarkers as well.

**DYSPHAGIA SYMPTOM QUESTIONNAIRE:** As used herein, "dysphagia symptom questionnaire" or "DSQ" refers to a validated patient-reported outcome questionnaire that captures the presence and severity of dysphagia to solid food in patients with EoE. Subjects record whether they have eaten solid food since waking up, any dysphagia to solid food on that day, dysphagia severity based on relief strategies utilized during the dysphagia episode and the severity of the worst daily pain related to swallowing, if there is any. The DSQ can be used to characterize baseline dysphagia severity and changes with treatment intervention. According to some embodiments, the DSQ total score range is 0 to 84.

**PHARMACEUTICAL COMPOSITION:** As used herein, "pharmaceutical composition" means a composition comprising at least one active ingredient, such as Compound 1, including but not limited to, salts, solvates, and hydrates of Compound 1, whereby the composition is amenable to investigation for a specified, efficacious outcome. Those of ordinary skill in the art will understand and appreciate the techniques appropriate for determining whether an active ingredient has a desired efficacious outcome based upon the needs of the artisan.

**HYDRATE:** As used herein, "hydrate" means a compound of the invention or a salt thereof, that further includes a stoichiometric or non-stoichiometric amount of water bound by non-covalent intermolecular forces.

**SOLVATE:** As used herein, "solvate" means a compound of the invention or a salt, thereof, that further includes a stoichiometric or non-stoichiometric amount of a solvent bound by non-covalent intermolecular forces. Preferred solvents are volatile, non-toxic, and/or acceptable for administration to humans in trace amounts.

Compound 1 may optionally be used as a pharmaceutically acceptable salt including a pharmaceutically acceptable acid addition salt prepared from pharmaceutically acceptable non-toxic acids including inorganic and organic acids. Representative acids include, but are not limited to, acetic, benzenesulfonic, benzoic, camphorsulfonic, citric, ethenesulfonic, dichloroacetic, formic, fumaric, gluconic, glutamic, hippuric, hydrobromic, hydrochloric, isethionic, lactic, maleic, malic, mandelic, methanesulfonic, mucic, nitric, oxalic, pamoic, pantothenic, phosphoric, succinic, sulfiric, tartaric, oxalic, *p*-toluenesulfonic and the like, such as those pharmaceutically acceptable salts listed by Berge et al., Journal of PharmaceuticalSciences, 66:1-19 (1977).

Acid addition salts may be obtained as the direct products of compound synthesis. In the alternative, the free base may be dissolved in a suitable solvent containing the appropriate acid and the salt isolated by evaporating the solvent or otherwise separating the salt and solvent. Compound 1 may form solvates with standard low molecular weight solvents using methods known to the skilled artisan.

It is understood that when the phrase "pharmaceutically acceptable salts, solvates and hydrates" or the phrase "pharmaceutically acceptable salt, solvate, or hydrate" is used when referring to Compound 1, it embraces pharmaceutically acceptable solvates and/or hydrates of Compound 1, pharmaceutically acceptable salts of Compound 1, as well as pharmaceutically acceptable solvates and/or hydrates of pharmaceutically acceptable salts of Compound 1. It is also understood that when the phrase "pharmaceutically acceptable solvates and hydrates" or the phrase "pharmaceutically acceptable solvate or hydrate" is used when referring to Compound 1that are salts, it embraces pharmaceutically acceptable solvates and/or hydrates of such salts. It will be apparent to those skilled in the art that the dosage forms described herein may comprise, as the active component, either Compound 1 or a pharmaceutically acceptable salt or as a solvate or hydrate thereof. Moreover, various hydrates and solvates of Compound 1 and their salts will find use as intermediates in the manufacture of pharmaceutical compositions. Typical procedures for making and identifying suitable hydrates and solvates, outside those mentioned herein, are well known to those in the art; see for example, pages 202-209 of K.J. Guillory, "Generation of Polymorphs, Hydrates, Solvates, and Amorphous Solids," in: Polymorphism in Pharmaceutical Solids, ed. Harry G. Britain, Vol. 95, Marcel Dekker, Inc., New York, 1999. Accordingly, one aspect of the present disclosure pertains to methods of prescribing and/or administering hydrates and solvates of Compound 1 and/or its pharmaceutical acceptable salts, that can be isolated and characterized by methods known in the art, such as, thermogravimetric analysis (TGA), TGA-mass spectroscopy, TGA-Infrared spectroscopy, powder X-ray diffraction (XRPD), Karl Fisher titration, high resolution X-ray diffraction, and the like. There are several commercial entities that provide quick and efficient services for identifying solvates and hydrates on a routine basis. Example companies offering these services include Wilmington PharmaTech (Wilmington, DE), Avantium Technologies (Amsterdam) and Aptuit (Greenwich, CT).

When an integer is used in a method disclosed herein, the term "about" can be inserted before the integer.

Throughout this specification, unless the context requires otherwise, the word "comprise", or variations such as "comprises" or "comprising" will be understood to imply the inclusion of a stated step or element or integer or group of steps or elements or integers but not the exclusion of any other step or element or integer or group of elements or integers.

Throughout this specification, unless specifically stated otherwise or the context requires otherwise, reference to a single step, composition of matter, group of steps, or group of compositions of matter shall be taken to encompass one and a plurality (*i.e.,* one or more) of those steps, compositions of matter, groups of steps, or groups of compositions of matter.

It is appreciated that certain features of the invention(s) described herein, which are, for clarity, described in the context of separate embodiments, can also be provided in combination in a single embodiment. Conversely, various features of the invention(s), which are, for brevity, described in the context of a single embodiment, can also be provided separately or in any suitable subcombination. For example, a method that recites prescribing and/or administering Compound 1 or a pharmaceutically acceptable salt, solvate, or hydrate thereof can be separated into two methods; one method reciting prescribing Compound 1 or a pharmaceutically acceptable salt, solvate, or hydrate thereof and the other method reciting administering Compound 1 or a pharmaceutically acceptable salt, solvate, or hydrate thereof. In addition, for example, a method that recites prescribing Compound 1 or a pharmaceutically acceptable salt, solvate, or hydrate thereof and a separate method of the invention reciting administering Compound 1 or a pharmaceutically acceptable salt, solvate, or hydrate thereof can be combined into a single method reciting prescribing and/or administering Compound 1 or a pharmaceutically acceptable salt, solvate, or hydrate thereof.

Provided is a compound that is (*R*)-2-(7-(4-cyclopentyl-3-(trifluoromethyl)benzyloxy)-1,2,3,4-tetrahydrocyclopenta[*b*]indol-3-yl)acetic acid (Compound 1), or a pharmaceutically acceptable salt thereof, for use in a method of treatment of eosinophilic esophagitis (EoE) in an individual, wherein the method comprises administering to an individual in need thereof a therapeutically effective amount of the compound or salt thereof.

In some embodiments, the individual is selected on the basis of exhibiting ≥15 eosinophils per high powered field (hpf) in the esophagus prior to or at the time of the treatment ("baseline").

In some embodiments, the individual exhibits at least 50% decrease in the number of eosinophils per hpf from baseline at day 10 following the administration of Compound 1, or a pharmaceutically acceptable salt thereof.

In some embodiments, the individual is selected on the basis of exhibiting an eotaxin-3 level of greater than about 50 pg/mL prior to or at the time of initiation of treatment ("baseline").

In some embodiments, the individual exhibits at least 50% decrease in eotaxin-3 level from baseline at day 10 following the administration.

In some embodiments, the EOE is diagnosed by endoscopy of the esophagus followed by microscopic and biochemical analysis of the esophageal mucosal lining. In some embodiment, the EOE is diagnosed by endoscopy of the esophagus and the presence and severity of esophageal endoscopic features (eg, edema, rings, exudates, furrows, and strictures). In some embodiments, the EOE is diagnosed by endoscopy and biopsy of one or more esophageal level (proximal, mid and/or distal). In some embodiments, the diagnosis is a result of the and analysis of the individual's eosinophil count and/or histologic characterization.

In some embodiments, the individual is susceptible to an allergen. For example, the subject may exhibit one of the following characteristics: (a) is prone to allergic reactions or responses when exposed to one or more allergens; (b) has previously exhibited an allergic response or reaction to one or more allergens; (c) has a known history of allergies; and/or (d) exhibits a sign or symptom of an allergic response or anaphylaxis. In certain embodiments, the subject is allergic to an allergen associated with EoE or that renders the subject susceptible and/or prone to developing EoE.

In some embodiments, the individual exhibits an allergic reaction to a food allergen. For example, the subject may have an allergy to an allergen contained in a food item including, but not limited to, a dairy product, egg, wheat, soy, corn, rye, fish, shellfish, peanut, a tree nut, beef, chicken, oat, barley, pork, green beans, and fruits such as apple and pineapple.

In some embodiments, the individual is allergic to a non-food allergen such as allergens derived from dust, mold, insects, plants including pollen, and pets such as cats and dogs. Examples of non-food allergens (also known as environmental allergens or aeroallergens) include, but are not limited to, house dust mite allergens, pollen allergens, animal dander allergens, insect venom, grass allergens, and latex.

In some embodiments, the symptom or indication of EoE is selected from eosinophilic infiltration of the esophagus, thickening of the esophageal wall, food refusal, vomiting, abdominal pain, heartburn, regurgitation, dysphagia and food impaction.

In some embodiments, the individual, prior to treatment, exhibits (or have exhibited) one or more indications of EoE such as, e.g., esophageal overexpression of pro-inflammatory mediators such as mast cells, eosinophilic infiltration of the esophagus, thickening of the esophageal wall, dysphagia, food impaction and chest and abdominal pain and/or an elevated level of an EoE-associated biomarker.

In some embodiments, the individual is more susceptible to EoE or may show an elevated level of an EoE-associated biomarker. For example, the individual is suffering from an atopic disease or disorder such as food allergy, atopic dermatitis, asthma, allergic rhinitis and allergic conjunctivitis. In some embodiments, the subject has an inherited connective tissue disorder. Such a subject population may show an elevated level of an EoE-associated biomarker such as, e.g., IgE, eotaxin-3, periostin, IL-5, or IL-13.

In some embodiments, the individual shows elevated levels of one or more EoE-associated biomarkers. In some embodiments, the administration of Compound 1, or a pharmaceutically acceptable salt thereof, results in reducing the level of an EoE-associated biomarker in the individual. In some embodiments, the EoE-associated biomarker is selected from esophagus eosinophils, eotaxin-3, periostin, serum IgE (total and allergen-specific), IL-13, IL-5, serum thymus and activation regulated chemokine (TARC), thymic stromal lymphopoietin (TSLP), serum eosinophilic cationic protein (ECP), and eosinophil-derived neurotoxin (EDN).

In some embodiments, prior to treatment, the individual shows the presence of ≥15 eosinophils per high power field in the esophagus. In some embodiments, prior to treatment, the individual shows an elevated peripheral eosinophil counts (>300 cells/up or elevated serum IgE (>150 kU/L). In some embodiments, prior to treatment, the individual shows the presence of ≥15 eosinophils per high power field in the esophagus and an elevated peripheral eosinophil counts (>300 cells/up or elevated serum IgE (>150 kU/L).

In some embodiments, Compound 1, or a pharmaceutically acceptable salt thereof reduces migration of tissue dendritic cells to a lymph node.

In some embodiments, Compound 1, or a pharmaceutically acceptable salt thereof reduces infiltrating TH2 and CD8 T cells. In some embodiments, Compound 1, or a pharmaceutically acceptable salt thereof reduces circulating T cells.

In some embodiments, Compound 1, or a pharmaceutically acceptable salt thereof reduces tissue cytokines.

In some embodiments, Compound 1, or a pharmaceutically acceptable salt thereof reduces eosinophil infiltration.

In some embodiments, Compound 1, or a pharmaceutically acceptable salt thereof, reduces eosinophil tissue accumulation.

In some embodiments, the individual, prior to or at the time of administration of Compound 1, or a pharmaceutically acceptable salt thereof, has or is diagnosed with a disease or disorder selected from atopic dermatitis, asthma, allergic rhinitis and allergic conjunctivitis.

In some embodiments, the pharmaceutical dosage form is administered once daily to the individual.

In some embodiments, the individual is administered an amount equivalent to about 0.5 to about 5.0 mg of Compound 1. In some embodiments, the individual is administered an amount equivalent to 2 mg of Compound 1. In some embodiments, the individual is administered an amount equivalent to 2.25 mg of Compound 1. In some embodiments, the individual is administered an amount equivalent to 2.5 mg of Compound 1. In some embodiments, the individual is administered an amount equivalent to 2.75 mg of Compound 1. In some embodiments, the individual is administered an amount equivalent to 3 mg of Compound 1. In some embodiments, the individual is administered an amount equivalent to 1 mg of Compound 1.

In some embodiments, the individual is administered Compound 1 or a pharmaceutically acceptable salt thereof at least one month, such as one month, two months, three months, four months, etc. In some embodiments, the individual is administered Compound 1 or a pharmaceutically acceptable salt thereof for least one week, such as one week, two weeks, three weeks, four weeks, five weeks, six weeks, seven weeks, eight weeks, nine weeks, ten weeks, eleven weeks, twelve weeks, thirteen weeks, fourteen weeks, fifteen weeks, sixteen weeks, etc. In some embodiments, the second time period is indefinite, e.g., chronic administration.

In some embodiments, the individual is administered an amount equivalent to 2 mg of Compound 1 for a first time period and subsequently an amount equivalent to 3 mg of Compound 1 for a second time period. In some embodiments, the first time period is at least one month, such as one month, two months, three months, four months, etc. In some embodiments, the first time period is at least one week, such as one week, two weeks, three weeks, four weeks, five weeks, six weeks, seven weeks, eight weeks, nine weeks, ten weeks, eleven weeks, twelve weeks, thirteen weeks, fourteen weeks, fifteen weeks, sixteen weeks, etc. In some embodiments, the second time period is at least one month, such as one month, two months, three months, four months, etc. In some embodiments, the second time period is at least one week, such as one week, two weeks, three weeks, four weeks, five weeks, six weeks, seven weeks, eight weeks, nine weeks, ten weeks, eleven weeks, twelve weeks, thirteen weeks, fourteen weeks, fifteen weeks, sixteen weeks, etc. In some embodiments, the second time period is indefinite, e.g., chronic administration.

In some embodiments, the individual is administered an amount equivalent to 3 mg of Compound 1 for a first time period and subsequently an amount equivalent to 2 mg of Compound 1 for a second time period. In some embodiments, the first time period is at least one month, such as one month, two months, three months, four months, etc. In some embodiments, the first time period is at least one week, such as one week, two weeks, three weeks, four weeks, five weeks, six weeks, seven weeks, eight weeks, nine weeks, ten weeks, eleven weeks, twelve weeks, thirteen weeks, fourteen weeks, fifteen weeks, sixteen weeks, etc. In some embodiments, the second time period is at least one month, such as one month, two months, three months, four months, etc. In some embodiments, the second time period is at least one week, such as one week, two weeks, three weeks, four weeks, five weeks, six weeks, seven weeks, eight weeks, nine weeks, ten weeks, eleven weeks, twelve weeks, thirteen weeks, fourteen weeks, fifteen weeks, sixteen weeks, etc. In some embodiments, the second time period is indefinite, e.g., chronic administration.

In some embodiments, the individual is administered an amount equivalent to 1 mg of Compound 1 for a first time period and subsequently an amount equivalent to 2 mg of Compound 1 for a second time period. In some embodiments, the first time period is at least one month, such as one month, two months, three months, four months, etc. In some embodiments, the first time period is at least one week, such as one week, two weeks, three weeks, four weeks, five weeks, six weeks, seven weeks, eight weeks, nine weeks, ten weeks, eleven weeks, twelve weeks, thirteen weeks, fourteen weeks, fifteen weeks, sixteen weeks, etc. In some embodiments, the second time period is at least one month, such as one month, two months, three months, four months, etc. In some embodiments, the second time period is at least one week, such as one week, two weeks, three weeks, four weeks, five weeks, six weeks, seven weeks, eight weeks, nine weeks, ten weeks, eleven weeks, twelve weeks, thirteen weeks, fourteen weeks, fifteen weeks, sixteen weeks, etc. In some embodiments, the second time period is indefinite, e.g., chronic administration.

In some embodiments, the individual is administered an amount equivalent to 2 mg of Compound 1 for a first time period and subsequently an amount equivalent to 1 mg of Compound 1 for a second time period. In some embodiments, the first time period is at least one month, such as one month, two months, three months, four months, etc. In some embodiments, the first time period is at least one week, such as one week, two weeks, three weeks, four weeks, five weeks, six weeks, seven weeks, eight weeks, nine weeks, ten weeks, eleven weeks, twelve weeks, thirteen weeks, fourteen weeks, fifteen weeks, sixteen weeks, etc. In some embodiments, the second time period is at least one month, such as one month, two months, three months, four months, etc. In some embodiments, the second time period is at least one week, such as one week, two weeks, three weeks, four weeks, five weeks, six weeks, seven weeks, eight weeks, nine weeks, ten weeks, eleven weeks, twelve weeks, thirteen weeks, fourteen weeks, fifteen weeks, sixteen weeks, etc. In some embodiments, the second time period is indefinite, e.g., chronic administration.

In some embodiments, the standard dose is administered without titration. In some embodiments, the standard dose is administered without titration; and the individual does not experience a severe related adverse event. In some embodiments, the standard dose is administered without requiring titration to avoid first-dose effect seen with other S1P receptor modulators.

In some embodiments, the dosage form is administered under fasted conditions. In some embodiments, the dosage form is administered under fed conditions.

In some embodiments, the method is non-gender specific.

In some embodiments, Compound 1, or a pharmaceutically acceptable salt thereof, is administered in combination with a second therapeutic agent or therapy, wherein the second therapeutic agent or therapy is selected from an IL-1beta inhibitor, an IL-5 inhibitor, an IL-9 inhibitor, an IL-13 inhibitor, an IL-17 inhibitor, an IL-25 inhibitor, a TNFα inhibitor, an eotaxin-3 inhibitor, an IgE inhibitor, a prostaglandin D2 inhibitor, an immunosuppressant, a glucocorticoid, a proton pump inhibitor, a NSAID, allergen removal and diet management.

In some embodiments, Compound 1, or a pharmaceutically acceptable salt thereof, was previously administered at least one therapeutic agent or therapy, wherein the therapeutic agent or therapy is selected from an IL-1beta inhibitor, an IL-5 inhibitor, an IL-9 inhibitor, an IL-13 inhibitor, an IL-17 inhibitor, an IL-25 inhibitor, a TNFα inhibitor, an eotaxin-3 inhibitor, an IgE inhibitor, a prostaglandin D2 inhibitor, an immunosuppressant, a glucocorticoid, a proton pump inhibitor, a NSAID, allergen removal and diet management.

In some embodiments, the individual is, or was, treated with an IL-1beta inhibitor. In some embodiments, the IL-1beta inhibitor is anakinra, rilonacept, or canakinumab.

In some embodiments, the individual is, or was, treated with an IL-5 inhibitor. In some embodiments, the IL-5 inhibitor is benralizumab, mepolizumab or reslizumab.

In some embodiments, the individual is treated with an IL-9 inhibitor.

In some embodiments, the individual is, or was, treated with an IL-13 inhibitor. In some embodiments, the IL-13 inhibitor is lebrikizumab, RPC4046, or tralokinumab.

In some embodiments, the individual is, or was, treated with an IL-17 inhibitor. In some embodiments, the IL-17 inhibitor is ixekizumab or brodalumab.

In some embodiments, the individual is, or was, treated with an IL-25 inhibitor.

In some embodiments, the individual is, or was, treated with a TNFα inhibitor. In some embodiments, the TNFα inhibitor is SIMPONI^{®} (golimumab), REMICADE^{®} (infliximab), HUMIRA^{®} (adalimumab), or CIMZIA^{®} (certolizumab pegol).

In some embodiments, the individual is, or was, treated with an eotaxin-3 inhibitor.

In some embodiments, the individual is, or was, treated with an IgE inhibitor. In some embodiments, the IgE inhibitor is omalizumab.

In some embodiments, the individual is, or was, treated with a prostaglandin D2 inhibitor.

In some embodiments, the individual is, or was, treated with an immunosuppressant. In some embodiments, the immunosuppressant is AZASAN^{®} (azathioprine), IMURAN^{®} (azathioprine), GENGRAF^{®} (cyclosporine), NEORAL^{®} (cyclosporine), or SANDIMMUNE^{®} (cyclosporine). Immunosuppressants also may be referred to as immunosuppressives or immunosuppressive agents.

In some embodiments, the individual is, or was, treated with a proton pump inhibitor. In some embodiments, the proton pump inhibitor is omeprazole, pantoprazole, esomeprazole, or dexlansoprazole.

In some embodiments, the individual is, or was, treated with a glucocorticoid. In some embodiments, the glucocorticoid is UCERIS^{®} (budesonide); DELTASONE^{®} (prednisone), MEDROL^{®} (methylprednisolone), or hydrocortisone. Glucocorticosteroids also may be referred to as glucocorticoid or corticosteroids.

In some embodiments, the individual is, or was, treated with a NSAID. In some embodiments, the NSAID is aspirin, celecoxib, diclofenac, diflunisal, etodolac, ibuprofen, indomethacin, ketoprofen, ketorolac, nabumetone, naproxen, oxaprozin, piroxicam, salsalate, sulindac, or tolmetin.

In some embodiments, the individual is, or was, treated with allergen removal.

In some embodiments, the individual is, or was, treated with diet management. In some embodiments, diet management comprises targeted elimination diets wherein foods that test positive on allergy testing or history are removed from the diet.

In some embodiments, diet management comprises empiric six-food elimination diet wherein instead of basing dietary elimination on allergy testing results, patients eliminate common allergy-causing foods (milk, eggs, wheat, soy, peanuts/tree nuts, fish/shellfish).

In some embodiments, diet management comprises an elemental diet wherein all sources of protein are removed from the diet and the patient drinks only an amino acid formula.

In some embodiments, diet management comprises food trial wherein specific foods are removed from the diet, and then added back, one at a time, to determine which food(s) cause a reaction. Diet management may involve repeat endoscopies with biopsies as foods are reintroduced to determine which foods are tolerated.

In some embodiments, the individual has had an inadequate response with, lost response to, been intolerant to, or demonstrated dependence on another agent for the treatment of eosinophilic esophagitis. In some embodiments, the individual has had an inadequate response with the other agent for the treatment of eosinophilic esophagitis. In some embodiments, the individual has lost response to another agent for the treatment of eosinophilic esophagitis. In some embodiments, the individual was intolerant to another agent for the treatment of eosinophilic esophagitis.

In some embodiments, the individual has had an inadequate response with, lost response to, or been intolerant to a conventional therapy. In some embodiments, the individual has had an inadequate response to conventional therapy. In some embodiments, the individual has lost response to conventional therapy. In some embodiments, the individual has been intolerant to conventional therapy. In some embodiments, the conventional therapy is selected from: an IL-1beta inhibitor, an IL-5 inhibitor, an IL-9 inhibitor, an IL-13 inhibitor, an IL-17 inhibitor, an IL-25 inhibitor, a TNFα inhibitor, an eotaxin-3 inhibitor, an IgE inhibitor, a prostaglandin D2 inhibitor, an immunosuppressant, a glucocorticoid, a proton pump inhibitor, a NSAID, allergen removal and diet management. In some embodiments, the prior conventional therapy is referred to as prior treatment.

In some embodiments, the individual had an inadequate response with, lost response to, or was intolerant to the at least one therapeutic agent or therapy. In some embodiments, the individual has demonstrated an inadequate response to, loss of response to, or intolerance of to the at least one therapeutic agent or therapy. In some embodiments, the individual had demonstrated, over the previous 3-month period, an inadequate response to, loss of response to, or intolerance of the at least one therapeutic agent or therapy. In some embodiments, the individual had demonstrated, over the previous 6-month period, an inadequate response to, loss of response to, or intolerance of the at least one therapeutic agent or therapy. In some embodiments, the individual had demonstrated, over the previous 9-month period, an inadequate response to, loss of response to, or intolerance of the at least one therapeutic agent or therapy. In some embodiments, the individual had demonstrated, over the previous 1-year period, an inadequate response to, loss of response to, or intolerance of the at least one therapeutic agent or therapy. In some embodiments, the individual had demonstrated, over the previous 2-year period, an inadequate response to, loss of response to, or intolerance of the at least one therapeutic agent or therapy. In some embodiments, the individual had demonstrated, over the previous 3-year period, an inadequate response to, loss of response to, or intolerance of the at least one therapeutic agent or therapy. In some embodiments, the individual had demonstrated, over the previous 4-year period, an inadequate response to, loss of response to, or intolerance of the at least one therapeutic agent or therapy. In some embodiments, the individual had demonstrated, over the previous 5-year period, an inadequate response to, loss of response to, or intolerance of the at least one therapeutic agent or therapy.

In some embodiments, prior to the treatment, the individual will have been administered proton pump inhibitor therapy. In some embodiments, prior to the treatment, the individual had an inadequate response with, lost response to, or was intolerant to proton pump inhibitor therapy. In some embodiments, the individual will have been on a stable dose of proton pump inhibitor therapy for at least two months.

In some embodiments, prior to the treatment, the individual will not have severe strictures.

In some embodiments, the method further comprises monitoring for adverse events during the administration of Compound 1, or a pharmaceutically acceptable salt thereof, and optionally, interrupting or terminating the administration of Compound 1, or a pharmaceutically acceptable salt thereof.

In some embodiments, the treatment further comprises monitoring heart rate during the administration, monitoring pulmonary function during the administration, or monitoring liver function during the administration.

In some embodiments, the treatment further comprises monitoring heart rate during the administration.

In some embodiments, the treatment further comprises monitoring pulmonary function during the administration.

In some embodiments, the treatment further comprises monitoring liver function during the administration.

In some embodiments, the method reduces the incidence and severity of adverse events resulting from the treatment of a condition described herein.

In some embodiments, the adverse event is a serious adverse event.

In some embodiments, the serious adverse event is selected from leukopenia, constipation, diarrhea, nausea, abdominal pain, neutropenia, vomiting, back pain, and menstrual disorder.

In some embodiments, the method results in no serious adverse events.

In some embodiments, the standard dose is administered without substantially inducing an acute heart rate reduction or heart block in the individual.

In some embodiments, Compound 1, or a pharmaceutically acceptable salt thereof, is administered without causing a reduction of more than 6 bpm in heart rate.

In some embodiments, Compound 1, or a pharmaceutically acceptable salt thereof, is administered without a first-dose effect on heart rate as seen with other S1P receptor modulators. In some embodiments, Compound 1, or a pharmaceutically acceptable salt thereof, is administered without a first-dose effect on AV conduction as seen with other S1P receptor modulators.

In some embodiments, the method of treatment is for improving endoscopic response. In some embodiments, the method of treatment is for endoscopic improvement, e.g., improving endoscopic appearance of the mucosa.

In some embodiments, treating comprises inducing and/or maintaining clinical response; improving endoscopic appearance of the mucosa; and/or inducing and/or maintaining clinical remission.

In some embodiments, treating comprises at least a 30% decrease in the number of eosinophils per hpf from baseline in a patient diagnosed with EOE. In some embodiments, treating comprises at least a 10%, 20%, 40%, 50%, 60%, 70%, 80%, 90% or 100% decrease in the number of eosinophils per hpf from baseline in a patient diagnosed with EOE. According to some embodiments, the decrease in the number of eosinophils per hpf from baseline in a patient diagnosed with EOE is achieved in 16 weeks. According to some embodiments, the decrease in the number of eosinophils per hpf from baseline in a patient diagnosed with EOE is achieved in 10, 12, 14, 16, 20, 24, 26, 30, 36, 40, or 52 weeks.

In some embodiments, treating comprises at least a 30% decrease in esophageal peak eosinophil count (PEC) from baseline in a patient diagnosed with EOE. In some embodiments, treating comprises at least a 10%, 20%, 40%, 50%, 60%, 70%, 80%, 90% or 100% decrease in esophageal PEC from baseline in a patient diagnosed with EOE. According to some embodiments, the decrease in the esophageal PEC from baseline in a patient diagnosed with EOE is achieved in 16 weeks. According to some embodiments, the decrease in the in the esophageal PEC from baseline in a patient diagnosed with EOE is achieved in 10, 12, 14, 16, 20, 24, 26, 30, 36, 40, or 52 weeks.

In some embodiments, treating comprises an improvement from baseline in Dysphasia Symptom Questionnaire (DSQ) score in a patient diagnosed with EOE. In some embodiments, treating comprises an improvement in DSQ score of at least 10 points, at least 20 points, at least 30 points, and the like.

In some embodiments, the treatment is for inducing clinical remission. In some embodiments, the treatment is for maintaining clinical remission. In some embodiments, the treatment is for inducing and maintaining clinical remission.

In some embodiments, the treatment is for inducing clinical response. In some embodiments, the treatment is for maintaining clinical response. In some embodiments, the treatment is for inducing and maintaining clinical response.

In some embodiments, the treatment is for endoscopic remission.

In some embodiments, treating is reducing a sign and/or symptom of eosinophilic esophagitis. In some embodiments, treating is reducing a sign of eosinophilic esophagitis. In some embodiments, treating is reducing a symptom of eosinophilic esophagitis. In some embodiments, treating comprises inducing and/or maintaining a histologic response via eosinophils per high power field (hpf) ≤6.

In some embodiments, treating is inducing and/or maintaining clinical remission. In some embodiments, treating is inducing and maintaining clinical remission. In some embodiments, treating is inducing and/or maintaining clinical remission and/or clinical response. In some embodiments, treating is inducing and maintaining clinical remission and clinical response. In some embodiments, treating is inducing clinical remission and/or clinical response. In some embodiments, treating is maintaining clinical remission and/or clinical response. In some embodiments, treating is inducing clinical remission and clinical response. In some embodiments, treating is maintaining clinical remission and clinical response. In some embodiments, treating is reducing signs and/or symptoms of eosinophilic esophagitis. In some embodiments, treating is reducing signs and symptoms of eosinophilic esophagitis. In some embodiments, treating is reducing signs of eosinophilic esophagitis. In some embodiments, treating is reducing symptoms of eosinophilic esophagitis. In some embodiments, treating is reducing signs and symptoms and inducing and maintaining clinical remission of eosinophilic esophagitis. In some embodiments, treating is reducing symptoms of eosinophilic esophagitis. In some embodiments, treating is reducing signs and symptoms and inducing and maintaining clinical remission of eosinophilic esophagitis in an individual who has had inadequate response to conventional therapy. In some embodiments, treating is reducing signs and symptoms and inducing and maintaining clinical remission of eosinophilic esophagitis in an individual who has lost response to or is intolerant to a conventional therapy. In some embodiments, treating is reducing signs and symptoms and inducing and maintaining clinical response in an individual with eosinophilic esophagitis who has had inadequate response to conventional therapy. In some embodiments, treating is reducing signs and symptoms and inducing and maintaining clinical response in an individual with eosinophilic esophagitis who has lost response to or is intolerant to a conventional therapy. In some embodiments, treating is inducing and/or maintaining clinical remission and/or mucosal healing. In some embodiments, treating is inducing and maintaining clinical remission and mucosal healing. In some embodiments, treating is inducing and maintaining mucosal healing. In some embodiments, treating is inducing and maintaining clinical remission. In some embodiments, treating is inducing clinical remission. In some embodiments, treating is inducing mucosal healing. In some embodiments, treating is maintaining clinical remission. In some embodiments, treating is maintaining mucosal healing. In some embodiments, treating is achieving and/or sustaining clinical remission in induction responders. In some embodiments, treating is achieving and sustaining clinical remission in induction responders. In some embodiments, treating is achieving clinical remission in induction responders. In some embodiments, treating is sustaining clinical remission in induction responders. In some embodiments, treating is inducing and/or maintaining clinical response. In some embodiments, treating is inducing and maintaining clinical response. In some embodiments, treating is inducing clinical response. In some embodiments, treating is maintaining clinical response. In some embodiments, treating is inducing endoscopic improvement. In some embodiments, treating is maintaining endoscopic improvement. In some embodiments, treating is achieved endoscopic improvement. In some embodiments, treating is improving endoscopic remission. In some embodiments, treating is maintaining endoscopic remission. In some embodiments, treating is inducing histologic healing. In some embodiments, treating is maintaining histologic healing. In some embodiments, treating is improving stool frequency. In some embodiments, treating is maintaining improvement in stool frequency. In some embodiments, treating is improving endoscopic appearance of the mucosa. In some embodiments, treating is maintaining endoscopic improvement of the mucosa. In some embodiments, treating is improving endoscopic appearance of the mucosa during induction. In some embodiments, treating is improving endoscopic subscore. In some embodiments, treating is maintaining improvement in endoscopic subscore.

In some embodiments, Compound 1, or a pharmaceutically acceptable salt thereof, is not recommended in an individual with active, severe infection. In some embodiments, Compound 1, or a pharmaceutically acceptable salt thereof, is not recommended in an individual with an active infection. In some embodiments, Compound 1, or a pharmaceutically acceptable salt thereof, is not recommended in an individual with a severe infection. In some embodiments, Compound 1, or a pharmaceutically acceptable salt thereof, is not recommended in an individual with an active, severe infection until the infection is controlled. In some embodiments, Compound 1, or a pharmaceutically acceptable salt thereof, is not recommended in an individual with an active infection until the infection is controlled. In some embodiments, Compound 1, or a pharmaceutically acceptable salt thereof, is not recommended in an individual with a severe infection until the infection is controlled. In some embodiments, administration of Compound 1, or a pharmaceutically acceptable salt thereof, is not started during an active infection. In some embodiments, an individual is monitored for infection. In some embodiments, administration of Compound 1, or a pharmaceutically acceptable salt thereof, is stopped if an individual develops an infection. In some embodiments, administration of Compound 1, or a pharmaceutically acceptable salt thereof, is stopped if infection becomes serious. In some embodiments, administration of Compound 1, or a pharmaceutically acceptable salt thereof, is discontinued if an individual develops an infection. In some embodiments, Compound 1, or a pharmaceutically acceptable salt thereof, is not administered to an individual with an infection. In some embodiments, Compound 1, or a pharmaceutically acceptable salt thereof, is not administered during an active infection. In some embodiments, administration of Compound 1, or a pharmaceutically acceptable salt thereof, is not started during active infection; an individual is monitored if an infection develops during administration; and administration is stopped if the infection becomes serious. In some embodiments, an infection is mild. In some embodiments, an infection is moderate. In some embodiments, an infection is severe. In some embodiments, an infection is serious. In some embodiments, an infection is a serious adverse event. In some embodiments, an infection is a respiratory infection.

In some embodiments, Compound 1, or a pharmaceutically acceptable salt thereof, is administered without causing a severe adverse event. In some embodiments, Compound 1, or a pharmaceutically acceptable salt thereof, is administered without causing a severe adverse event related to heart rate. In some embodiments, Compound 1, or a pharmaceutically acceptable salt thereof, is administered without causing a severe adverse event related to heart rate change. In some embodiments, Compound 1, or a pharmaceutically acceptable salt thereof, is administered without causing a severe adverse event related to elevated heart rate. In some embodiments, Compound 1, or a pharmaceutically acceptable salt thereof, is administered without causing a severe adverse event related to bradycardia. In some embodiments, Compound 1, or a pharmaceutically acceptable salt thereof, is administered without causing a severe adverse event related to AV block. In some embodiments, Compound 1, or a pharmaceutically acceptable salt thereof, is administered without causing a severe adverse event related to AV conduction. In some embodiments, Compound 1, or a pharmaceutically acceptable salt thereof, is administered without causing bradycardia. In some embodiments, Compound 1, or a pharmaceutically acceptable salt thereof, is administered without causing AV block. In some embodiments, Compound 1, or a pharmaceutically acceptable salt thereof, is administered without causing more than mild decrease in heart rate on first day of treatment (for example, >10 bpm). In some embodiments, Compound 1, or a pharmaceutically acceptable salt thereof, is administered without a first-dose effect seen with other S1P receptor modulators. In some embodiments, Compound 1, or a pharmaceutically acceptable salt thereof, is administered without a first-dose cardiovascular effect seen with other S1P receptor modulators. In some embodiments, Compound 1, or a pharmaceutically acceptable salt thereof, is administered without symptomatic changes in heart rate. In some embodiments, Compound 1, or a pharmaceutically acceptable salt thereof, is administered without symptomatic changes in heart rhythm. In some embodiments, Compound 1, or a pharmaceutically acceptable salt thereof, is administered without requiring titration to avoid first-dose effect seen with other S1P receptor modulators.

In some embodiments, Compound 1, or a pharmaceutically acceptable salt thereof, is administered without increasing a liver function test (LFT). In some embodiments, Compound 1, or a pharmaceutically acceptable salt thereof, is administered without causing an elevated LFT. In some embodiments, Compound 1, or a pharmaceutically acceptable salt thereof, is administered without increasing ALT. In some embodiments, Compound 1, or a pharmaceutically acceptable salt thereof, is administered without increasing AST. In some embodiments, Compound 1, or a pharmaceutically acceptable salt thereof, is administered without increasing ALT >3X ULN. In some embodiments, Compound 1, or a pharmaceutically acceptable salt thereof, is administered without increasing ALT >2.5X ULN. In some embodiments, Compound 1, or a pharmaceutically acceptable salt thereof, is administered without increasing ALT >2X ULN. In some embodiments, Compound 1, or a pharmaceutically acceptable salt thereof, is administered without increasing ALT >1.5X ULN. In some embodiments, Compound 1, or a pharmaceutically acceptable salt thereof, is administered without increasing AST >3X ULN. In some embodiments, Compound 1, or a pharmaceutically acceptable salt thereof, is administered without increasing AST >2.5X ULN. In some embodiments, Compound 1, or a pharmaceutically acceptable salt thereof, is administered without increasing AST >2X ULN. In some embodiments, Compound 1, or a pharmaceutically acceptable salt thereof, is administered without increasing AST >1.5X ULN. In some embodiments, Compound 1, or a pharmaceutically acceptable salt thereof, is administered without increasing bilirubin. In some embodiments, Compound 1, or a pharmaceutically acceptable salt thereof, is administered without increasing bilirubin >3X ULN. In some embodiments, Compound 1, or a pharmaceutically acceptable salt thereof, is administered without increasing bilirubin >2.5X ULN. In some embodiments, Compound 1, or a pharmaceutically acceptable salt thereof, is administered without increasing bilirubin >2X ULN. In some embodiments, Compound 1, or a pharmaceutically acceptable salt thereof, is administered without increasing bilirubin >1.5X ULN. In some embodiments, Compound 1, or a pharmaceutically acceptable salt thereof, is administered without increasing gamma-glutamyl transferase (GGT). In some embodiments, Compound 1, or a pharmaceutically acceptable salt thereof, is administered without increasing GGT >3X ULN. In some embodiments, Compound 1, or a pharmaceutically acceptable salt thereof, is administered without increasing GGT >2.5X ULN. In some embodiments, Compound 1, or a pharmaceutically acceptable salt thereof, is administered without increasing GGT >2X ULN. In some embodiments, Compound 1, or a pharmaceutically acceptable salt thereof, is administered without increasing GGT >1.5X ULN.

In some embodiments, Compound 1, or a pharmaceutically acceptable salt thereof, is administered without causing an abnormality in a pulmonary function test. In some embodiments, Compound 1, or a pharmaceutically acceptable salt thereof, is administered without causing macular edema.

In some embodiments, the Compound 1, or a pharmaceutically acceptable salt thereof, is administered orally.

In some embodiments, the Compound 1, or a pharmaceutically acceptable salt thereof, is formulated as a capsule or tablet suitable for oral administration.

In some embodiments, the Compound 1, or a pharmaceutically acceptable salt thereof, is selected from: Compound 1; a calcium salt of Compound 1; and an L-arginine salt of Compound 1. In some embodiments, the Compound 1, or a pharmaceutically acceptable salt thereof, is an L-arginine salt of Compound 1. In some embodiments, the Compound 1, or a pharmaceutically acceptable salt thereof, is an anhydrous, non-solvated crystalline form of an L-arginine salt of Compound 1. In some embodiments, the Compound 1, or a pharmaceutically acceptable salt thereof, is an anhydrous, non-solvated crystalline form of Compound 1.

Also provided are pharmaceutical compositions comprising a standard dose of Compound 1, or, a pharmaceutically acceptable salt, a hydrate or solvate thereof and, optionally, one or more pharmaceutically acceptable carriers. Also provided are pharmaceutical compositions comprising Compound 1, or, a pharmaceutically acceptable salt, a hydrate or solvate thereof, optionally, one or more pharmaceutically acceptable carriers. The carrier(s) must be "acceptable" in the sense of being compatible with the other ingredients of the formulation and not overly deleterious to the recipient thereof.

In some embodiments, Compound 1, or, a pharmaceutically acceptable salt, a hydrate or solvate thereof, is administered as a raw or pure chemical, for example as a powder in capsule formulation.

In some embodiments, Compound 1, or, a pharmaceutically acceptable salt, a hydrate or solvate thereof, is formulated as a pharmaceutical composition further comprising one or more pharmaceutically acceptable carriers.

Pharmaceutical compositions may be prepared by any suitable method, typically by uniformly mixing the active compound(s) with liquids or finely divided solid carriers, or both, in the required proportions and then, if necessary, forming the resulting mixture into a desired shape.

Conventional excipients, such as binding agents, fillers, acceptable wetting agents, tabletting lubricants and disintegrants may be used in tablets and capsules for oral administration. The compounds described herein can be formulated into pharmaceutical compositions using techniques well known to those in the art. Suitable pharmaceutically acceptable carriers, outside those mentioned herein, are known in the art; for example, see Remington, The Science and Practice of Pharmacy, 20th Edition, 2000, Lippincott Williams & Wilkins, (Editors: Gennaro *et al*.)

For oral administration, the pharmaceutical composition may be in the form of, for example, a tablet or capsule. The pharmaceutical composition is preferably made in the form of a dosage unit containing a particular amount of the active ingredient. Examples of such dosage units are capsules, tablets, powders, granules or suspensions, with conventional additives such as lactose, mannitol, corn starch or potato starch; with binders such as crystalline cellulose, cellulose derivatives, acacia, corn starch or gelatins; with disintegrators such as corn starch, potato starch or sodium carboxymethyl-cellulose; and with lubricants such as talc or magnesium stearate. Solid form preparations include powders, tablets, pills, capsules, cachets, suppositories, and dispersible granules. A solid carrier can be one or more substances which may also act as diluents, flavoring agents, solubilizers, lubricants, suspending agents, binders, preservatives, tablet disintegrating agents, or encapsulating materials.

In powders, the carrier is a finely divided solid which is in a mixture with the finely divided active component.

In tablets, the active component is mixed with the carrier having the necessary binding capacity in suitable proportions and compacted to the desired shape and size.

The powders and tablets may contain varying percentage amounts of the active compound. A representative amount in a powder or tablet may be from 0.5 to about 90 percent of the active compound. However, an artisan would know when amounts outside of this range are necessary. Suitable carriers for powders and tablets include magnesium carbonate, magnesium stearate, talc, sugar, lactose, pectin, dextrin, starch, gelatin, tragacanth, methylcellulose, sodium carboxymethyl cellulose, a low melting wax, cocoa butter, and the like. The term "preparation" includes the formulation of the active compound with encapsulating material as carrier providing a capsule in which the active component, with or without carriers, is surrounded by a carrier, which is thus in association with it. Similarly, cachets and lozenges are included. Tablets, powders, capsules, pills, cachets, and lozenges can be used as solid forms suitable for oral administration.

The pharmaceutical preparations are preferably in unit dosage forms. In such form, the preparation is subdivided into unit doses containing appropriate quantities of the active component. The unit dosage form can be a packaged preparation, the package containing discrete quantities of preparation, such as packeted tablets or capsules. Also, the unit dosage form can be a capsule or tablet itself, or it can be the appropriate number of any of these in packaged form.

Further embodiments include the embodiments disclosed in the following Examples.

### EXAMPLES

### EXAMPLE 1

Formulations composed of immediate-release, hard gelatin capsules containing an L-arginine salt of Compound 1 were prepared as shown in **Table 1.**

**Table 1**

| | **Formulation** | | | | |
|---|---|---|---|---|---|
| | **0.1 mg** | **0.35 mg** | **0.5 mg** | **1 mg** | **2 mg** |
| L-arginine salt of Compound 1 (mg/capsule) | 0.14 | 0.48 | 0.69 | 1.38 | 2.76 |
| Empty capsule weight (mg)* | 38.0 | 61.0 | 61.0 | 61.0 | 61.0 |
| Total capsule target weight (mg)** | 38.14 | 61.48 | 61.69 | 62.38 | 63.76 |

| | | | | | |
|---|---|---|---|---|---|
| *Approximate weight. Based on capsule specification **Theoretical total weight calculated by combining fill and empty capsule weights together | | | | | |

### EXAMPLE 2

Formulations composed of immediate-release tablets containing an L-arginine salt of Compound 1 were prepared as shown in **Table 2.**

**Table 2**

| **Tablet Strength** | **0.5 mg** | **1 mg** | **2 mg** | **3 mg** |
|---|---|---|---|---|
| L-Arg Salt of Compound 1 | 0.69 | 1.381 | 2.762 | 4.143 |
| Mannitol Pearlitol^{®} 100SD | 54.81 | 54.119 | 52.738 | 51.357 |
| Microcrystalline cellulose - | 40 | 40 | 40 | 40 |
| Sodium Starch Glycolate - | 4 | 4 | 4 | 4 |
| Magnesium Stearate | 0.5 | 0.5 | 0.5 | 0.5 |
| Opadry^{®} II Blue | 4 | 4 | 4 | 4 |
| Total tablet target weight | 104 | 104 | 104 | 104 |

### EXAMPLE 3

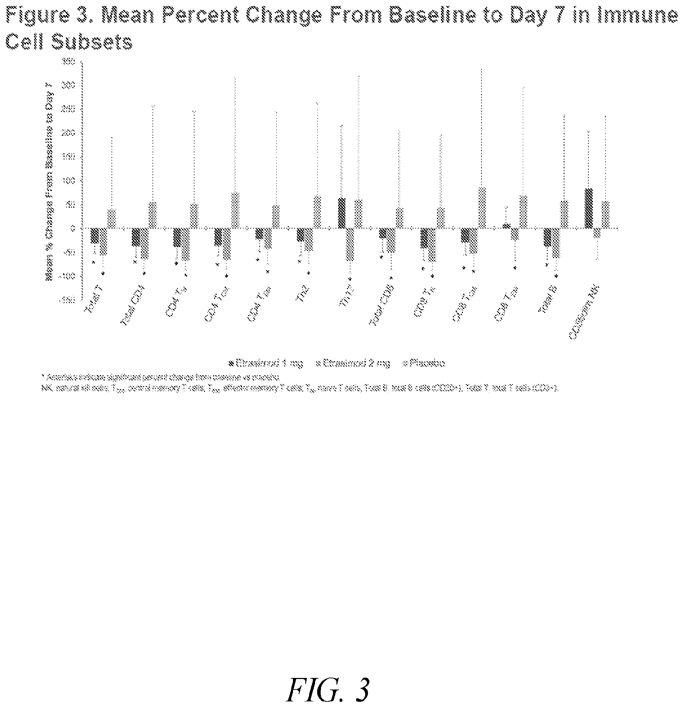

A single-blind (ie, subject only), placebo-controlled, randomized, fixed-sequence, parallel design study evaluated PK, PD, safety, and tolerability in healthy Japanese and Caucasian male subjects (data reported herein is based on the preliminary clinical study report). A total of 49 subjects were enrolled and received 1 or 2 mg of Compound 1 or placebo once daily for 7 days followed by an additional single dose on Day 15, administered 1 week after a treatment-free period. Progressive decreases in median lymphocyte count were observed in all treatment groups following the first dose (as determined by the first complete blood count measurement following the first dose on Day 2) through the end of the repeated dosing period on Day 8. The median percent change from baseline at Day 7 was - 23.61% in the Japanese 1 mg group, -25.79% in the Caucasian 1 mg group, -50.00% in the Japanese 2 mg group, and -43.67% in the Caucasian 2 mg group. The median percent change from baseline at Day 15 (pre-dose) was 0.00% in the Japanese 1 mg group, -21.43% in the Caucasian 1 mg group, -6.67% in the Japanese 2 mg group, and -16.23% in the Caucasian 2 mg group. In depth immunophenotyping was performed in healthy subjects. In general, subjects treated with Compound 1 showed a dose-dependent decrease in the median number of circulating B cells, CD4+ TCM, CD4+ TN, Th17, Th2, CD8+ TCM, and CD8+ TN cells, which peaked at Day 7 pre-dose. This data is shown in FIGURE 3. This study helped demonstrate that Compound 1 induces a dose-dependent decrease in total lymphocyte count in healthy subjects, including Th2 T cells which are associated with EoE pathogenesis.

### EXAMPLE 4

An ovalbumin (OVA)-induced mouse model of EGD will be evaluated. Treatment groups will include the following: 1.) naive (n=5); 2.) OVA challenge only (n=10); 3.) OVA challenge + dexamethasone PO (n=10); 4.) OVA challenge + vehicle control PO (n=10); 5.) OVA challenge + 1 mg/kg Compound 1 PO (n=10); and 6.) OVA challenge + 3 mg/kg Compound 1 PO (n=10). Compound 1 and vehicle will be administered twice daily, while dexamethasone will be administered once daily on challenge days.

Female Balb/c mice will be sensitized intraperitoneally (IP) on day 0 and 14 with 50 µg of OVA + 1 mg of aluminum hydroxide adjuvant in phosphate buffered saline (PBS). The mice will subsequently be challenged by oral gavage three times/week for three weeks with 10 mg OVA suspended in 100 µl PBS.

Esophageal tissue, stomach and intestine tissue, and blood/serum will be collected. Esophageal, stomach, and intestine samples will be evaluated for eosinophils by immunohistochemistry (IHC) using anti-mouse Major Basic Protein antibody. Esophageal lysates or serum will be analyzed for expression of the following cytokines/chemokines: eotaxin, G-CSF, GM-CSF, INFg, IL-1a, IL-1b, IL-2, IL-3, IL-4, IL-5, IL-6, IL-7, IL-9, IL-10, IL-12 (p40), IL-12 (p70), IL-13, IL-15, IL-17A, IP-10, KC, LIF, LIX, MCP-1, M-CSF, MIG, MIP-1a, MIP1b, MIP-2, RANTES, TNFa, and VEGF-A.

Blood/serum will be collected and aliquoted for flow cytometry, PK, anti-OVA IgE and cytokine analysis. Total cell count and cell differentiation will be carried out by FACS analysis for CD45, CD3, TCRb, TCRgd, CD25, CD11c, MHC-II, CD103, CD4, CD8, B220/CD19, Siglec F as well as viability. Levels of anti-OVA IgE antibody will measured via ELISA, and serum will be evaluated for PK.

The mice that received Compound 1 exhibited a dose-dependent decrease in lymphocyte levels.

### EXAMPLE 5

The use of Compound 1 in an L2-IL5^{OXA} mouse model of EOE will be evaluated. A mouse model of oesophageal inflammation will be utilized using a 4-ethoxymethylene-2-phenyl-2-oxazolin-5-one (OXA; Sigma, St Louis, Missouri, USA) contact hypersensitivity protocol (as described in Masterson JC, McNamee EN, Hosford L, Capocelli KE, Ruybal J, Fillon SA, Doyle AD, Eltzschig HK, Rustgi AK, Protheroe CA, Lee NA, Lee JJ, Furuta GT. Local hypersensitivity reaction in transgenic mice with squamous epithelial IL-5 overexpression provides a novel model of eosinophilic oesophagitis. Gut. 2014 Jan;63(1):43-53.

Different groups of L2-IL5 mice will be used in each experiment. Treatment groups include the following: 1.) vehicle; 2.) OXA challenge only; 3.) vehicle challenge + 1 mg/kg Compound 1 PO ; 4.) OXA challenge + 2 mg/kg Compound 1 PO; 5.) OXA challenge + 3 mg/kg Compound 1 PO; and 6.) OXA challenge + 10 mg/kg dexamethasone IP.

On day 0 of the OXA hypersensitivity protocol, abdominal skin of anaesthetized experimental animals will be shaved and OXA will be applied to the skin surface (3% (w/v) solution of OXA in vehicle) to initiate the sensitization phase of the protocol. Sensitized mice will receive topical OXA challenges of the oesophagus by gavage (p.c.) on protocol days 5, 8 and 12 with a 1% (w/v) solution of OXA in 30% vehicle into the proximal oesophagus. Vehicle control animals will be sensitized as noted above and challenged with 4:1 vehicle alone. All mice will be assessed 24 h following the last OXA challenge (protocol day 13). Additional groups of mice will be concurrently treated with the corticosteroid dexamethasone (DEX) by intraperitoneal (i.p.) injection of mice (10 mg/kg of body weight) or an oral solution of Compound 1 during the topical OXA challenge phase (protocol days 5, 8, 10 and 12 for DEX, daily or twice daily for Compound 1); control animals will receive intraperitoneal injections of saline vehicle alone.

Esophageal tissue, stomach and intestine tissue, and blood/serum will be collected and analyzed. Oesophagi and blood serum will be assessed for cytokine production. Tissue samples will be evaluated for eosinophils. Esophageal lysates or serum will be analyzed for expression of one or more of the following cytokines/chemokines: eotaxin, G-CSF, GM-CSF, INFg, IL-1a, IL-1b, IL-2, IL-3, IL-4, IL-5, IL-6, IL-7, IL-9, IL-10, IL-12 (p40), IL-12 (p70), IL-13, IL-15, IL-17A, IFN-g, IP-10, KC, LIF, LIX, MCP-1, M-CSF, MIG, MIP-1a, MIP1b, MIP-2, RANTES, TNFa, and VEGF-A.

Blood/serum will be collected and aliquoted for flow cytometry, PK, and cytokine analysis. Total cell count and cell differentiation will be carried out by FACS analysis for one or more of CD45, CD3, TCRb, TCRgd, CD25, CD11b, CD11c, MHC-II, CD103, CD4, CD8, CD19, Siglec F as well as viability.

### EXAMPLE 6

A clinical trial following the FDA Guidance on EoE Drug Development (Draft, Feb. 2019) will be conducted. The trial population will have histologic confirmation via EGD: eosinophils per high power field (eos/hpf) of ≥15. Biopsies will be taken from both proximal and distal esophagus, and may include mid-esophagus for additional information. Individuals will have failed proton pump inhibitor (PPI) therapy or can be on stable dose for at least 2 months with histological confirmation. The trial will exclude individuals with severe strictures and will stratify randomization by presence or absence of baseline strictures.

The trial will be designed with a study duration for chronic treatment of at least 24 weeks to assess efficacy on both clinical and histological endpoints, followed by extension to provide total treatment period of at least 52 weeks. The trial will include a randomized withdrawal design following initial assessment of efficacy to characterize persistence of treatment and incidence of relapse and need for redosing.

The endpoints will include (1) improvement from base line in signs and symptoms using well-defined clinical outcome assessments and optionally will include anchor (e.g., patient global impression scale), supplemented with empirical cumulative 165 distribution functions using data pooled across groups; and (2) histologic response via eos/hpf ≤6.

### EXAMPLE 7

A phase 2 randomized, double-blind, placebo-controlled clinical trial will be conducted to assess safety, efficacy, and PK of Compound 1 in individuals with eosinophilic esophagitis. The trial will include the following: 1.) a screening period to perform esophagogastroduodenoscopy (EGD) and biopsies, 2.) a 16-week double-blind induction treatment period with 1 mg Compound 1, 2 mg Compound 1, or placebo, and 3.) an extension period.

Eligibility criteria will include histologic confirmation of EoE (e.g., eos/hpf ≥15 in 2 of 3 segments); demonstrated dysphagia (e.g., 2 episodes/week x 2 weeks); and a 4-week washout of topical steroids. Primary and secondary outcomes will include: histology endpoints from proximal, mid, and distal biopsies of the esophagus; % change in peak eos/hpf (e.g., at week 16), proportion achieving eos/hpf ≤6 (and <15) at week 16; change in EoE histologic scoring system (HSS) score and grade; % change in patient-reported outcomes (e.g., % change in Dysphagia Symptom Questionnaire (DSQ)), change in eosinophilic esophagitis activity index (EEsAI), and change in endoscopic reference score (EREFS).

### EXAMPLE 8

A phase 2/3 randomized, double-blind, placebo-controlled clinical trial will be conducted in individuals with histologic confirmation of EoE (eos/hpf ≥15), demonstrated dysphagia, and insufficient response to a proton pump inhibitor (PPI). The trial will include the following: 1.) a dose-finding period of up to 24 weeks with Dose 1 of Compound 1, Dose 2 of Compound 1, or placebo 2.) a pivotal period of up 24 weeks with the selected dose of Compound 1 or placebo, and 3.) an extension period with the selected dose of Compound 1 or placebo. A follow-up visit will subsequently be conducted.

Proximal and distal biopsies of the esophagus will be performed. Endpoints will include the changes in eos/hpf (e.g., % achieving eos/hpf ≤6) and responses to a dysphagia symptom questionnaire.

### EXAMPLE 9

A Phase 2, randomized, double-blind, multi-center study will evaluate the efficacy, safety, and pharmacokinetics (PK) of Compound 1 compared with placebo in adults with active EoE. The study will consist of a screening period of up to 28 days, 24 weeks of double-blind treatment (Double-Blind Treatment Period), 28 weeks of active extended treatment (Extension Treatment Period), and 4 weeks of follow-up (Safety Follow-Up Period) for a total study duration of up to 60 weeks.

Eligible subjects will be randomized in a double-blind fashion (3:3:2 ratio) to receive oral tablet of 1 mg of Compound 1, 2 mg of Compound 1, or matching placebo once daily during the Double-Blind Treatment Period and the Extension Treatment Period. The formulations provided of 1 mg or 2 mg of Compound 1 may be prepared as described in Example 1. One tablet is to be taken once daily with water (either with or without food) at approximately the same time each day, preferably in the morning.

All subjects who complete the Double-Blind Treatment Period and meet eligibility criteria for the Extension Treatment Period may enter the Extension Treatment Period. Subjects who were in the 1 mg or 2 mg of Compound 1 groups in the Double-Blind Treatment Period will continue the same dose of Compound 1 in the Extension Treatment Period. Subjects who were in the placebo group during the Double-Blind Treatment Period will be re-randomized (1:1 ratio) to 1 mg or 2 mg of Compound 1 at entry into the Extension Treatment Period. Study treatment (1 mg or 2 mg of Compound 1) will remain blinded.

Following the Double-Blind Treatment Period dose selection analysis at Week 24, a dose of Compound 1 (1 mg or 2 mg) may be discontinued in the Extension Treatment Period. In this scenario, subjects receiving the discontinued dose in the Extension Treatment Period will be switched to the selected Compound 1 dose at the next study visit.

### Inclusion Criteria

- Men or women between 18 and 65 years of age at the time of informed consent (IC)
- Have histologically active EoE with an esophageal peak eosinophil count (PEC) of ≥ 15 eosinophils (eos)/high power field (hpf) (~60 eos/mm2) from any level (proximal, mid, or distal) of the esophagus at the screening esophagogastroduodenoscopy (EGD). Eosinophilia must be isolated to the esophagus.
- Have dysphagia, defined as solid food going down slowly or getting stuck in the throat with an average frequency of ≥ 2 episodes per week over 2 weeks (as documented using the Dysphagia Symptom Questionnaire (DSQ) during the Screening period)

### Exclusion Criteria

- History of any of the following non-EoE conditions or procedures that may interfere with the evaluation of or affect the histologic, endoscopic, or symptom endpoints of the study: a. Conditions that cause or potentially contribute to esophageal eosinophilia (eg, eosinophilic gastritis [EG], gastroenteritis, or colitis with esophageal involvement, severe gastroesophageal reflux disease [GERD], achalasia and other disorders of esophageal dysmotility, hypereosinophilic syndrome, Crohn's disease [CD] with esophageal involvement, esophageal infection [fungal, viral], connective tissue diseases, hypermobility syndromes, autoimmune disorders and vasculitides, dermatologic conditions with esophageal involvement [ie, pemphigus], drug hypersensitivity reactions, pill esophagitis, graft versus host disease, Mendelian disorders [eg, Marfan syndrome Type II, hyper-immunoglobulin E (IgE) syndrome, phosphatase and tensin homolog hamartoma tumor syndrome, Netherton syndrome, severe atopy metabolic wasting syndrome])
- b. Conditions that interfere with the evaluation of the esophagus (eg, esophageal varices with risk of spontaneous bleed, high-grade esophageal stenosis where an 8- to 10-mm endoscope could not pass through the stricture without dilation at the time of screening EGD)
- c. Conditions or procedures that cause or potentially contribute to dysphagia (eg, Barrett's esophagus, erosive esophagitis Los Angeles Grade B or above, significant hiatal hernia [≥ 4 cm], esophageal resection, fundoplication, gastric sleeve surgery)
- Undergone dilation of an esophageal stricture within 12 weeks prior to screening EGD.
- Use of corticosteroids for the treatment of EoE within 8 weeks prior to screening EGD.
- Discontinue, initiate, or change dosing (dosage/frequency) of the following therapies for EoE within 8 weeks prior to screening EGD. Subjects on any of the following therapy need to stay on a stable regimen during study participation:
   a. Elemental diet
   b. EoE food trigger elimination diet
   c. PPI therapy
- Used any immunotherapy/desensitization including oral immunotherapy (OIT) or sublingual immunotherapy (SLIT) within 12 months prior to the Screening EGD Note: Stable (ie, ≥ 6 months prior to the screening EGD) subcutaneous immunotherapy (SCIT) is permitted. Subjects on SCIT need to stay on a stable treatment during study participation.
- Used any of the following immunomodulatory therapies within the timeframes prior to baseline as indicated below. Within two weeks, antimetabolites (eg, AZA, 6-MP, MTX, 6-TG), calcineurin inhibitors (eg, cyclosporine, tacrolimus), MMF; within 12 weeks (Anti-IL-5 antibodies (eg, mepolizumab, reslizumab, benralizumab), anti-IL-4/13 antibodies (eg, dupilumab), anti-IgE antibodies (eg, omalizumab), TNFα inhibitors (eg, infliximab), JAK inhibitors (eg, tofacitinib, oclacitinib); within twenty four weeks (Anti-CD20 antibodies (eg, rituximab, ocrelizumab), anti-CD52 antibodies (eg, alemtuzumab), other cell-depleting therapies (eg, bone marrow transplantation, total body irradiation); or sphingosine 1-phosphate receptor modulators (eg, fingolimod, siponimod, ozanimod) or natalizumab taken at any time before baseline measurements are taken for the study.

Subjects will have follow-up visits at 2 and 4 weeks after the last dose of study treatment after Week 52 or the Early Termination (ET) Visit.

### Efficacy Endpoints:

Efficacy measures to be evaluated in this study include Esophageal peak eosinophil count (PEC), EoE Histology Scoring System (HSS), DSQ, Food Avoidance Question (FAQ); Eosinophilic Esophagitis Endoscopic Reference Score (EREFS), Adult Eosinophilic Esophagitis Quality of Life (EoE-QOL-A), the Patient Global Impression of Change (PGIC), and Patient Global Impression of Severity (PGIS).

### Primary efficacy endpoints include:

- Percent change from baseline in esophageal PEC at Week 16

### Secondary efficacy endpoints include:

- Absolute change from baseline in DSQ score at Week 16
- Absolute change from baseline in esophageal PEC at Week 16
- Proportion of subjects with esophageal PEC < 15 eos/hpf at Week 16
- Proportion of subjects with esophageal PEC ≤ 6 eos/hpf at Week 16

Safety will be assessed through the incidence of adverse events (AEs), clinical laboratory findings, 12-lead electrocardiograms (ECGs) (for first-dose monitoring, on treatment re-initiation after a defined period of treatment interruption, and at the start of the Extension Treatment Period, to be done predose and at 4 hours following the first dose), physical examinations, vital signs (measured hourly for at least 4 hours following the first dose), pulmonary function tests (PFTs), ophthalmoscopy and optical coherence tomography (OCT).

## Claims

1. A compound that is (R)-2-(7-(4-cyclopentyl-3-(trifluoromethyl)benzyloxy)-1,2,3,4-tetrahydrocyclopenta[b]indol-3-yl)acetic acid (Compound 1), or a pharmaceutically acceptable salt thereof, for use in a method of treatment of eosinophilic esophagitis (EoE) in an individual, wherein the method comprises:
administering to an individual in need thereof a therapeutically effective amount of (*R*)-2-(7-(4-cyclopentyl-3-(trifluoromethyl)benzyloxy)-1,2,3,4-tetrahydrocyclopenta[*b*]indol-3-yl)acetic acid (Compound 1), or a pharmaceutically acceptable salt thereof.

2. The compound or salt for use according to claim 1, wherein:
the therapeutically effective amount is equivalent to about 0.5 to about 5.0 mg of Compound 1.

3. The compound or salt for use according to claim 1, wherein:
the therapeutically effective amount is equivalent to 1 mg of Compound 1.

4. The compound or salt for use according to claim 1, wherein:
the therapeutically effective amount is equivalent to 2 mg of Compound 1.

5. The compound or salt for use according to claim 1, wherein:
the therapeutically effective amount is equivalent to 3 mg of Compound 1.

6. The compound or salt for use according to claim 1, wherein:
the individual is administered an amount equivalent to 2 mg of Compound 1 for a first time period and subsequently an amount equivalent to 3 mg of Compound 1 for a second time period.

7. The compound or salt for use according to any one of claims 1-8, wherein:
the therapeutically effective amount is administered at a frequency of once per day.

8. The compound or salt for use according to any one of claims 1-7, wherein:
the Compound 1, or a pharmaceutically acceptable salt thereof, is administered orally.

9. The compound or salt for use according to any one of claims 1-8, wherein:
the Compound 1, or a pharmaceutically acceptable salt thereof, is an L-arginine salt of Compound 1.

10. The compound or salt for use according to any one of claims 1-9, wherein:
the individual in need thereof exhibits ≥15 eosinophils per high powered field (hpf) in the individual's esophagus prior to or at the time of the treatment ("baseline").

11. The compound or salt for use according to any one of claims 1-10, wherein:
the individual exhibits at least 50% decrease in the number of eosinophils per hpf from baseline at day 10 following the administration of Compound 1, or a pharmaceutically acceptable salt thereof.

12. The compound or salt for use according to any one of claims 1-11, wherein:
the administration of Compound 1, or a pharmaceutically acceptable salt thereof, results in reducing the level of an EoE-associated biomarker in the individual.

13. The compound or salt for use according to claim 12, wherein:
the EoE-associated biomarker is selected from esophagus eosinophils, eotaxin-3, periostin, serum IgE (total and allergen-specific), IL-13, IL-5, serum thymus and activation regulated chemokine (TARC), thymic stromal lymphopoietin (TSLP), serum eosinophilic cationic protein (ECP), and eosinophil-derived neurotoxin (EDN).

## Patentansprüche

1. Verbindung, welche (R)-2-(7-(4-Cyclopentyl-3-(trifluormethyl)benzyloxy)-1,2,3,4-tetrahydrocyclopenta[b]indol-3-yl)essigsäure (Verbindung 1) oder ein pharmazeutisch annehmbares Salz davon ist, zur Verwendung in einem Verfahren zur Behandlung von eosinophiler Ösophagitis (EoE) bei einem Individuum, wobei das Verfahren umfasst:
Verabreichen einer therapeutisch wirksamen Menge von (*R*)-2-(7-(4-Cyclopentyl-3-(trifluormethyl)benzyloxy)-1,2,3,4-tetrahydrocyclopenta[*b*]indol-3-yl)essigsäure (Verbindung 1) oder eines pharmazeutisch annehmbaren Salzes davon an ein dafür bedürftiges Individuum.

2. Verbindung oder Salz zur Verwendung nach Anspruch 1, wobei:
die therapeutisch wirksame Menge etwa 0,5 bis etwa 5,0 mg der Verbindung 1 entspricht.

3. Verbindung oder Salz zur Verwendung nach Anspruch 1, wobei:
die therapeutisch wirksame Menge 1 mg der Verbindung 1 entspricht.

4. Verbindung oder Salz zur Verwendung nach Anspruch 1, wobei:
die therapeutisch wirksame Menge 2 mg der Verbindung 1 entspricht.

5. Verbindung oder Salz zur Verwendung nach Anspruch 1, wobei:
die therapeutisch wirksame Menge 3 mg der Verbindung 1 entspricht.

6. Verbindung oder Salz zur Verwendung nach Anspruch 1, wobei:
dem Individuum eine Menge, die 2 mg der Verbindung 1 entspricht, für einen ersten Zeitraum und anschließend eine Menge, die 3 mg der Verbindung 1 entspricht, für einen zweiten Zeitraum verabreicht wird.

7. Verbindung oder Salz zur Verwendung nach einem der Ansprüche 1-8, wobei:
die therapeutisch wirksame Menge mit einer Häufigkeit von einmal pro Tag verabreicht wird.

8. Verbindung oder Salz zur Verwendung nach einem der Ansprüche 1-7, wobei:
die Verbindung 1 oder ein pharmazeutisch annehmbares Salz davon oral verabreicht wird.

9. Verbindung oder Salz zur Verwendung nach einem der Ansprüche 1-8, wobei:
die Verbindung 1 oder ein pharmazeutisch annehmbares Salz davon ein L-Argininsalz von Verbindung 1 ist.

10. Verbindung oder Salz zur Verwendung nach einem der Ansprüche 1-9, wobei:
das dafür bedürftige Individuum ≥15 Eosinophile pro Hauptgesichtsfeld (HPF) in der Speiseröhre des Individuums vor oder zu dem Zeitpunkt der Behandlung (''Ausgangswert") aufweist.

11. Verbindung oder Salz zur Verwendung nach einem der Ansprüche 1-10, wobei:
das Individuum mindestens 50 % Abnahme der Anzahl von Eosinophilen pro HPF vom Ausgangswert an Tag 10 nach der Verabreichung von Verbindung 1, oder einem pharmazeutisch annehmbaren Salz davon, aufweist.

12. Verbindung oder Salz zur Verwendung nach einem der Ansprüche 1-11, wobei:
die Verabreichung von Verbindung 1 oder eines pharmazeutisch annehmbaren Salzes davon zu Reduzierung des Gehalts eines EoE-assoziierten Biomarkers in dem Individuum führt.

13. Verbindung oder Salz zur Verwendung nach Anspruch 12, wobei:
der EoE-assoziierte Biomarker ausgewählt ist aus Ösophagus-Eosinophilen, Eotaxin-3, Periostin, Serum-IgE (gesamt und allergenspezifisch), IL-13, IL-5, Serum-Thymus- und aktivierungsreguliertem Chemokin (TARC), Thymus-Stroma-Lymphopoietin (TSLP), Serum-eosinophilem kationischem Protein (ECP) und Eosinophil-abgeleitetem Neurotoxin (EDN).

## Revendications

1. Composé qui est de l'acide (R)-2-(7-(4-cyclopentyl-3-(trifluorométhyl)benzyloxy)-1,2,3,4-tétrahydrocyclopenta[b]indol-3-yl)acétique (Composé 1) ou un sel pharmaceutiquement acceptable de celui-ci, pour une utilisation dans un procédé de traitement de l'œsophagite à éosinophiles (EoE) chez un individu, dans lequel le procédé comprend :
l'administration à un individu en ayant besoin d'une quantité thérapeutiquement efficace d'acide (R)-2-(7-(4-cyclopentyl-3-(trifluorométhyl)benzyloxy)-1,2,3,4-tétrahydrocyclopenta[b]indol-3-yl)acétique (Composé 1) ou d'un sel pharmaceutiquement acceptable de celui-ci.

2. Composé ou sel pour une utilisation selon la revendication 1, dans lequel :
la quantité thérapeutiquement efficace est équivalente à environ 0,5 à environ 5,0 mg de Composé 1.

3. Composé ou sel pour une utilisation selon la revendication 1, dans lequel :
la quantité thérapeutiquement efficace est équivalente à 1 mg de Composé 1.

4. Composé ou sel pour une utilisation selon la revendication 1, dans lequel :
la quantité thérapeutiquement efficace est équivalente à 2 mg de Composé 1.

5. Composé ou sel pour une utilisation selon la revendication 1, dans lequel :
la quantité thérapeutiquement efficace est équivalente à 3 mg de Composé 1.

6. Composé ou sel pour une utilisation selon la revendication 1, dans lequel :
l'on administre à l'individu une quantité équivalente à 2 mg de Composé 1 pendant une première période de temps et par la suite une quantité équivalente à 3 mg de Composé 1 pendant une deuxième période de temps.

7. Composé ou sel pour une utilisation selon l'une quelconque des revendications 1 à 8, dans lequel :
la quantité thérapeutiquement efficace est administrée à une fréquence d'une fois par jour.

8. Composé ou sel pour une utilisation selon l'une quelconque des revendications 1 à 7, dans lequel :
le Composé 1, ou un sel pharmaceutiquement acceptable de celui-ci, est administré par voie orale.

9. Composé ou sel pour une utilisation selon l'une quelconque des revendications 1 à 8, dans lequel :
le Composé 1, ou un sel pharmaceutiquement acceptable de celui-ci, est un sel de L-arginine du Composé 1.

10. Composé ou sel pour une utilisation selon l'une quelconque des revendications 1 à 9, dans lequel :
l'individu en ayant besoin présente ≥ 15 éosinophiles par champ de haute puissance (hpf) dans l'œsophage de l'individu avant ou pendant le traitement (« niveau de référence ») .

11. Composé ou sel pour une utilisation selon l'une quelconque des revendications 1 à 10, dans lequel :
l'individu présente une diminution d'au moins 50 % du nombre d'éosinophiles par hpf par rapport au niveau de la référence au jour 10 suivant l'administration du Composé 1 ou d'un sel pharmaceutiquement acceptable de celui-ci.

12. Composé ou sel pour une utilisation selon l'une quelconque des revendications 1 à 11, dans lequel :
l'administration du Composé 1, ou d'un sel pharmaceutiquement acceptable de celui-ci, entraîne une réduction du niveau d'un biomarqueur associé à l'EoE chez l'individu.

13. Composé ou sel pour une utilisation selon la revendication 12, dans lequel :
le biomarqueur associé à l'EoE est choisi parmi les éosinophiles de l'œsophage, l'éotaxine 3, la périostine, les IgE sériques (totales et spécifiques aux allergènes), l'IL-13, l'IL-5, le thymus sérique et la chimiokine régulée par activation (TARC), la lymphopoïétine stromale thymique (TSLP), la protéine cationique éosinophile sérique (ECP) et la neurotoxine dérivée des éosinophiles (EDN).
